# FASCICULE DE BREVET EUROPEEN

(11) **EP 1 090 027 B1**
(45) Date de publication et mention de la délivrance du brevet: **16.04.2003**
(21) Numéro de dépôt: 99957166.4
(22) Date de dépôt: 22.06.1999
(51) Int. Cl.: C07J 41/00, A61K 31/56

(54) **NOUVEAUX 19-NOR STEROIDES 17-HALOGENES, PROCEDE ET INTERMEDIAIRES DE PREPARATION, APPLICATION COMME MEDICAMENTS ET COMPOSITIONS PHARMACEUTIQUES LES RENFERMANT**
NEUE 19-NOR 17-HALO STEROID VERBINDUNGEN, EIN VERFAHREN ZU IHRER HERSTELLUNG UND IHRE VERWENDUNG ALS ARTZNEIMITTELN SOWIE PHARMAZEUTISCHE PRÄPARATE DAVON
NOVEL 17-HALOGENATED 19-NOR STEROIDS, METHOD AND INTERMEDIATES FOR PREPARING SAME, USE AS MEDICINES AND PHARMACEUTICAL COMPOSITIONS CONTAINING SAME

(30) Priorité: 23.06.1998 FR 9807898
(43) Date de publication de la demande: 11.04.2001
(73) Titulaire: Aventis Pharma S.A., 92160 Antony (FR)
(72) Inventeur: BOUALI, Yasmina, F-94800 Villejuif (FR); MAUGER, Jacques, F-75011 Paris (FR); NIQUE, François, F-94170 Le Perreux sur Marne (FR); VAN DE VELDE, Patrick, F-75019 Paris (FR)
(74) Mandataire: Vieillefosse, Jean-Claude
(86) Numéro de dépôt international: FR9901491
(87) Numéro de publication internationale: WO99067274

(56) Documents cités:
- WO-A-98/28324
- WO-A-98/45316
- WO-A-99/25725
- FR-A- 2 640 977
- US-A- 3 079 408
- US-A- 3 275 623
- US-A- 3 413 321
- JIN L ET AL: "ANTISTROGENIC ACTIVITY OF TWO 11BETA-ESTRADIOL DERIVATIVES ON MCF -7 BREAST CANCER CELLS" STEROIDS: STRUCTURE, FUNCTION, AND REGULATION, vol. 60, no. 8, août 1995 (1995-08), pages 512-518, XP002040770
- ARUNACHALAM T ET AL: "Iodoestrogens, syntheses, and interaction with uterine receptors" JOURNAL OF BIOLOGICAL CHEMISTRY, vol. 254, no. 13, 10 juillet 1979 (1979-07-10), pages 5900-5905, XP002089838 MD US
- H. BRUNNER ET AL: "Synthese und Antitumoraktivität von cis-Dichloroplatin(II)-Komplexen mit Östradiolderivaten" MONATSHEFTE FUR CHEMIE., vol. 124, no. 1, 1993, pages 83-102, XP002089839 WIEN AT

## Description

La présente invention concerne des composés 19-nor stéroïdes 17-halogénés, leurs procédé et intermédiaires de préparation, leur application comme médicament et les compositions pharmaceutiques les renfermant.

L'ostéoporose est une pathologie qui se caractérise par une réduction quantitative et qualitative du tissu osseux, suffisante pour entraîner des fractures vertébrales ou périphériques, ce de façon spontanée ou à l'occasion de traumatisme minime. Bien que cette affection soit d'origine multifactorielle, c'est la ménopause qui, chez la femme, constitue le facteur prépondérant de la perte osseuse ou ostéopénie.

Cette ostéopénie se manifeste par une raréfaction et une modification de l'architecture de l'os spongieux qui a pour conséquence d'accentuer la fragilité squelettique et le risque fracturaire. La perte osseuse s'accentue fortement après la ménopause en raison de la suppression de la fonction ovarienne et atteint 3 à 5 % par an pour se ralentir après 65 ans.

Dans un but thérapeutique, la carence hormonale post ménopausique peut être compensée par une hormonothérapie substitutive où l'oestrogène joue un rôle majeur en préservant le capital osseux. Mais l'estrogénothérapie au long cours s'accompagne parfois d'effet indésirable sur l'appareil génital (hyperplasie endométriale, tumeur mammaire...), ce qui constitue un inconvénient majeur et limite son application.

Il convient donc de trouver d'autres composés que l'oestradiol ayant une activité oestrogène dissociée, à savoir une activité oestrogène au niveau osseux, tout en n'ayant pas ou peu d'activité d'hyperplasie endométriale, ni d'activité de prolifération de tumeur mammaire. FR 2,528,434, WO-A-99/25725 et WO-A-98/45316 décrivent des dérivés de 19-nor stéroïdes présentant des activités hormonales ou antihormonales qui diffèrent des composés selon l'invention au niveau de la substitution en position 17.

L'invention a donc pour objet les composés de formule générale (I) : 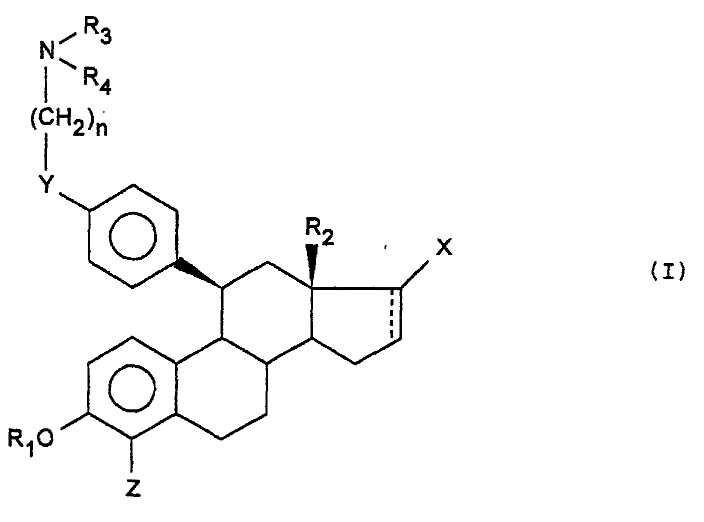 dans laquelle :
R₁ représente un atome d'hydrogène ou un radical choisi parmi méthyle, éthyle, COCH₃, COEt, COPh et CH₂Ph
R₂ représente un radical méthyle,
X représente un atome d'halogène,
Y représente une simple liaison, O, NH, S, SO ou SO₂
Z représente un atome d'hydrogène ou un atome d'halogène
n est égal à 2, 3, 4 ou 5,
soit R₃ et R₄ identiques ou différents représentent un atome d'hydrogène ou un groupement alkyle renfermant de 1 à 6 atomes de carbone, soit R₃ et R₄ forment ensemble avec l'atome d'azote auquel ils sont liés un hétérocycle mono ou polyclique, saturé, de 3 à 15 chaînons renfermant éventuellement de 1 à 3 hétéroatomes additionnels choisis parmi l'oxygène, le soufre et l'azote, non substitué ou substitué par un méthyle,
les traits en pointillés représentent une seconde liaison éventuelle, ainsi que leurs sels d'addition avec les bases ou les acides.

On entend par halogène : iode, brome, chlore ou fluor. En position 4, il s'agit de préférence de chlore ou brome. En position 17, il s'agit de préférence de fluor.

Lorsque R₃ et R₄ forment ensemble avec l'atome d'azote auquel ils sont liés un hétérocycle, il s'agit notamment des hétérocycles mono ou bicycliques renfermant éventuellement un autre hétéroatome choisi parmi l'oxygène et l'azote tels que les hétérocycles saturés suivants :

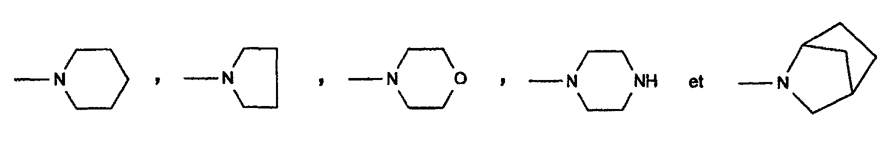

Bien entendu les valeurs de R₁, R₃ et R₄, ainsi que de n, sont indépendantes les unes des autres.

L'invention s'étend naturellement aux sels des composés de formule (I), comme par exemple les sels formés avec des acides minéraux ou organiques sur l'amine. Il peut alors s'agir des acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques. Lorsque les composés de formule (I) comportent une fonction acide, l'invention s'étend aux sels des métaux alcalins, alcalino terreux ou d'ammonium éventuellement substitués.

L'invention a plus particulièrement pour objet les composés de formule générale (I) telle que définie plus haut ainsi que leurs sels d'addition, dans laquelle X est un atome de fluor en position alpha, et les traits en pointillés ne représentent pas une seconde liaison (cycle D du stéroïde saturé).

L'invention a également plus particulièrement pour objet les composés de formule générale (I) telle que définie précédemment ainsi que leurs sels d'addition, dans laquelle R₁ est un atome d'hydrogène, R₂ est un radical méthyle et Z est soit un atome d'hydrogène, soit un atome de chlore, Y représente un atome d'oxygène, et les traits en pointillés ne représentent pas une seconde liaison.

L'invention a tout particulièrement pour objet les composés de formule générale (I) telle que définie précédemment ainsi que leurs sels d'addition, dans laquelle :
soit R₃ et R₄, identiques ou différents, représentent un radical alkyle renfermant de 1 à 6 atomes de carbone,
soit R₃ et R₄ forment ensemble avec l'atome d'azote auquel ils sont liés un des hétérocycles saturés suivants :

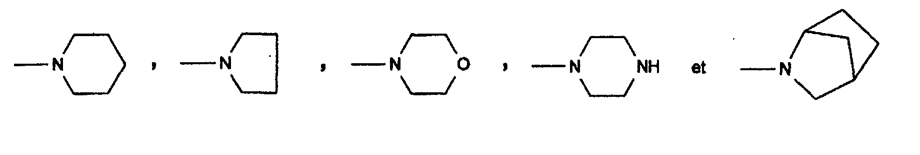

L'invention a également tout particulièrement pour objet les composés de formule générale (I) telle que définie précédemment ainsi que leurs sels d'addition dans laquelle X est un atome de fluor en position alpha, R₁ est un atome d'hydrogène, R₂ est un radical méthyle, Y est un atome d'oxygène, Z est un atome d'hydrogène ou un atome de chlore, n est égal à 2 ou 3,
soit R₃ et R₄, identiques ou différents, représentent un radical alkyle renfermant de 1 à 6 atomes de carbone,
soit R₃ et R₄ forment ensemble avec l'atome d'azote auquel ils sont liés un des hétérocycles saturés suivants : 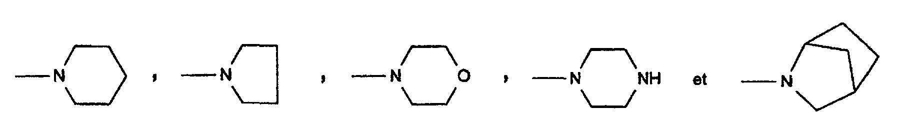 et les traits pointillés ne représentent pas une seconde liaison.

Enfin l'invention a pour objet les composés de formule (I) ainsi que leurs sels d'addition avec les acides dont les noms suivent :
- 17 alpha-fluoro-11 bêta-[4-[2-(1-pipéridinyl)éthoxy] phényl]-estra-1,3,5(10)-trièn-3-ol,
- Chlorhydrate de 17 alpha-fluoro-11-bêta-[4-[2-(1-pipéridinyl)éthoxy]phényl]-estra-1,3,5(10)-trièn-3-ol,
- 17 alpha-fluoro-11 bêta-[4-[2-(1-diéthylamino) éthoxy] phényl]-estra-1,3,5(10)-trièn-3-ol,
- 17 alpha-fluoro-11 beta-[4-[2-(1-pyrrolidinyl) éthoxy] phényl]-estra-1,3,5(10)-trièn-3-ol,
- 4-chloro-17 alpha-fluoro-11-bêta-[4-[2-(1-pipéridinyl) éthoxy]phényl]-estra-1,3,5(10)-trièn-3-ol,
- 17-iodo-11 bêta-[4-[2-(1-pipéridinyl)éthoxy]phényl]-estra-1,3,5(10),16-tétraèn-3-ol,
- 17alpha-fluoro-11bêta-[4-[2-(4-méthyl-1-pipéridinyl)-éthoxy]-phényl]-estra-1,3,5(10)-trien-3-ol,
- chlorhydrate de 17alpha-fluoro-11bêta-[4-[2-(4-méthyl-1-pipéridinyl)-éthoxy]-phényl]-estra-1,3,5(10)-trien-3-ol,
- 17alpha-fluoro-3-méthoxy-11bêta-[4-[2-(1-pipéridinyl)-éthoxy]-phényl]-estra-1,3,5(10)-triène,
- 17alpha-fluoro-3-méthoxy-11bêta-[4-[2-(1-pyrrolidinyl)-éthoxy]-phényl]-estra-1,3,5(10)-triène,
- 17alpha-fluoro-3-méthoxy-11bêta-[4-[2-(diéthylamino)-éthoxy]-phényl]-estra-1,3,5(10)-triène,
- (11bêta)-17chloro-11-[4-[2-(1-pipéridinyl)-éthoxy]-phényl]-estra-1,3,5(10),16-tétraèn-3-ol,
- 17alpha-chloro-11bêta-[4-[2-(1-pipéridinyl)-éthoxy]-phényl]-estra-1,3,5(10)-trièn-3-ol,
- chlorhydrate de 17-iodo-11bêta-[4-[2-(1-pipéridinyl)-éthoxy]-phényl]-estra-1,3,5(10),16-tétraèn-3-ol,
- lactate de 17alpha-fluoro-11bêta-[4-[2-(1-pipéridinyl)-éthoxy]-phényl]-estra-1,3,5(10)-trièn-3-ol.

L'invention a également pour objet un procédé de préparation des composés de formule (I) telle que définie précédemment, dans lequel on soumet un composé de formule (II) 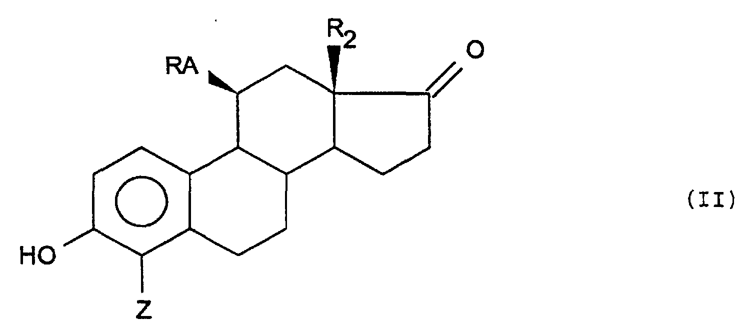 dans laquelle :
R₂ et Z sont tels que définis précédemment,
RA représente un des groupements suivants : 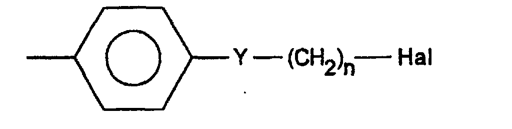 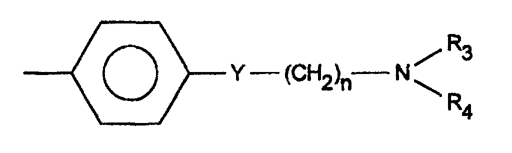 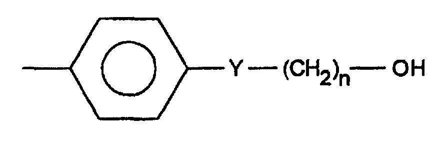 dans lesquels Y, n, R₃ et R₄ sont tels que définis précédemment et Hal représente un atome d'halogène, à l'action, après le cas échéant protection et/ou activation des fonctions OH,
soit a) d'un agent réducteur du céto en 17 afin d'obtenir un composé de formule (IIIₐ) : 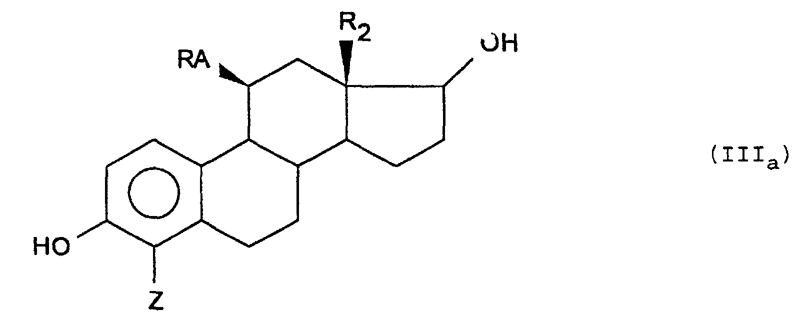
b) puis d'un agent d'halogénation afin d'obtenir un composé de formule (I'ₐ) : 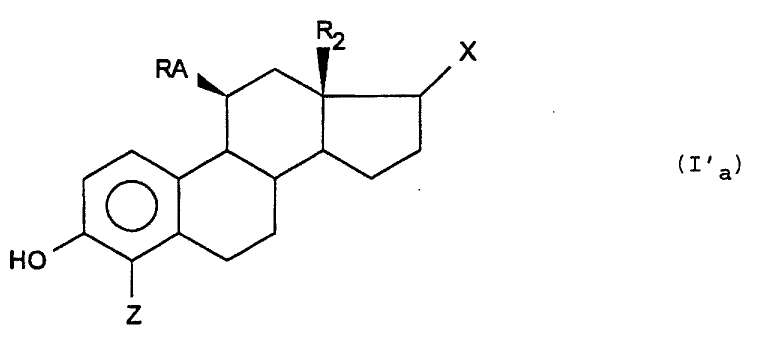
correspondant à certains composés de formule (I), lorsque RA représente -Ph-Y-(CH₂)ₙ-NR₃R₄,
soit a) d'une hydrazine afin d'obtenir un composé de formule (III_{b}) : 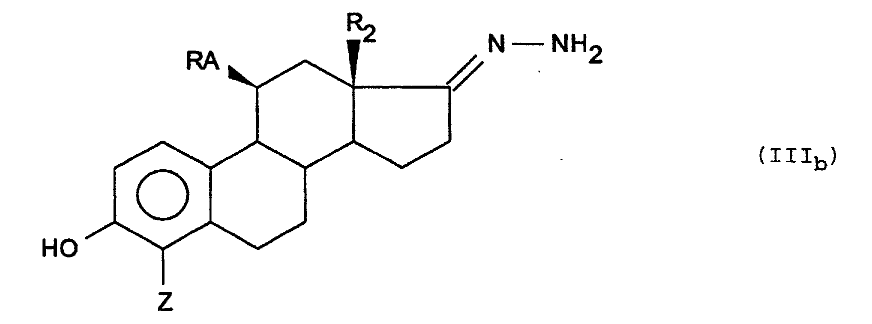
b) puis d'un agent d'halogénation afin d'obtenir un composé de formule (I'_{b}) : 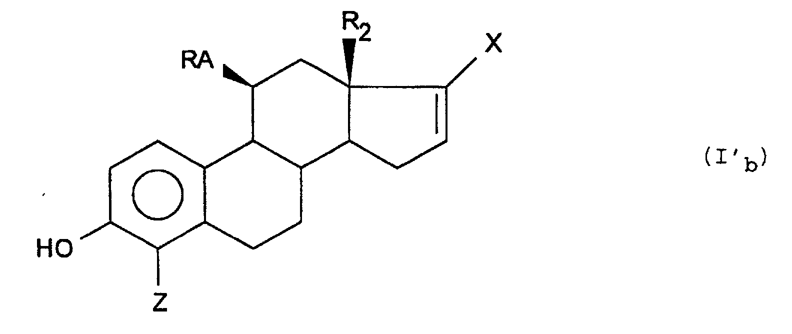
correspondant à certains composés de formule (I), lorsque RA représente -Ph-Y-(CH₂)ₙ-NR₃R₄,
les composés de formule (II), (IIIₐ), (III_{b}), (I'ₐ) ou (I'_{b}) sous forme protégée ou non, étant soumis si désiré ou si nécessaire, à l'une ou plusieurs des réactions suivantes :
- déprotection du ou des groupements OH protégés,
- acylation/alkylation du ou des groupements OH,
- action de HNR₃R₄, éventuellement sous forme de sel, lorsque RA représente le groupement -Ph-Y-(CH₂)ₙ-Hal ou -Ph-Y-(CH₂)ₙ-OH activé,
- salification.

La réduction du 17-céto en alcool s'effectue selon les méthodes classiques, notamment par action d'un borohydrure alcalin tel que le borohydrure de sodium dans le méthanol ou l'éthanol ou par action de tétrahydrure d'aluminium et de lithium.

Cette réduction permet d'obtenir en particulier l'alcool en position 17bêta.

La réaction d'halogénation qui suit s'effectue de préférence avec des réactifs tels que XSO₂C₄F₉ en présence d'une base encombrée telle que le DBU (diazabicycloundécène), X est de préférence le fluor. D'autres méthodes d'halogénation connues de l'homme de métier peuvent également être utilisées.

Lorsque le groupement hydroxy du produit de départ est en position bêta, on observe une inversion de la configuration lors de la substitution nucléophile et on obtient par ce procédé (réactif d'halogénation : fluorure de perfluoro-1-butane sulfonyle (FSO₂C₄F₉)) tout particulièrement les composés de formule (I) ou (I'ₐ) avec le fluor en position 17-alpha.

L'action de l'hydrazine s'effectue de préférence en présence d'une base telle que la triéthylamine et l'halogénation qui suit s'effectue notamment avec X₂ en milieu basique et notamment avec I₂.

On entend par activation de l'alcool, l'introduction notamment d'un mésylate, tosylate ou triflate qui permet de favoriser la substitution nucléophile de l'amine HNR₃R₄ sur les composés de formules (II), (IIIₐ), (III_{b}), (I'ₐ), (I'_{b}) dans lesquelles R₃ représente un groupement -Ph-Y-(CH₂)ₙ-OH.

La formation du mésylate, tosylate ou triflate à partir de l'alcool correspondant s'effectue en présence d'une base telle que la triéthylamine. On peut également préalablement envisager la substitution de l'alcool par un atome d'halogène selon les méthodes usuelles.

Les réactions de protection et de déprotection sont les méthodes classiques connues de l'homme du métier. Une revue assez complète se trouve dans l'ouvrage suivant : Protective groups in organic synthesis, T.W Greene, John Wiley & sons (1981).

Le groupement protecteur P (OH → OP) peut représenter un radical alkyle renfermant de 1 à 4 atomes de carbone, un groupement benzyle, un groupement tétrahydropyrannyle, un groupement R_{C}R_{D}R_{E}Si, dans lequel R_{C}, R_{D} et R_{E} identiques ou différents, indépendamment l'un de l'autre représentent chacun un radical alkyle renfermant de 1 à 4 atomes de carbone ou un groupement phényle. Il s'agit tout particulièrement des groupements Si(Me)₂CMe₃ ou -Si(Ph)₂CMe₃ ou -SiMe₃.

A titre d'exemple, les réactions de déprotection (OP --> OH en position 3), lorsque P est un groupement tertbutyldiphénylsilyle peuvent s'effectuer par action de fluorure de tétrabutyl ammonium en solution dans le tétrahydrofuranne. Il en est de même lorsque P représente SO₂C₄F₉ suite à la réaction de fluoration.

Lorsque P est un groupement tétrahydropyrannyle, la déprotection s'effectue en présence d'un acide aqueux dans un solvant alcoolique et de préférence par action de l'acide chlorhydrique dans le méthanol.

L'action d'un composé de formule R₃R₄NH sur les composés de formules (II), (IIIₐ), (III_{b}), (I'ₐ) ou (I'_{b}) avec R₂ représentant un groupement -Ph-Y-(CH₂)ₙ-OH ou -Ph-Y-(CH₂)ₙ-Hal s'effectue dans les conditions classiques des substitutions nucléophiles, notamment en présence d'un solvant aprotique tel que le tétrahydrofuranne. Lorsque OH est activé il s'agit notamment de OSO₂CH₃, OSO₂-Ph-pMe ou OSO₂CPh₃.

Les réactions d'alkylation ou d'acylation du groupement OH en position 3 ainsi que les réactions de salification s'effectuent selon les méthodes classiques connues de l'homme du métier.

Les composés de formule générale (I) ainsi que leurs sels d'addition avec les acides pharmaceutiquement acceptables possèdent notamment des activités oestrogène, anti-oestrogène et antiprolifératives.

A ce titre, les composés de formule (I) peuvent être utilisés dans le traitement des troubles liés à une hypofolliculinie, par exemple les aménorrhées, les dysménorrhées, les avortements répétés, les troubles prémenstruels, dans le traitement de certaines pathologies estrogèno-dépendantes telles que les adénomes ou les carcinomes prostatiques, les carcinomes mammaires et ses métastases ou le traitement des tumeurs bénignes du sein, en tant qu'anti-utérotrophique ainsi que dans le traitement substitutif de la ménopause ou de la périménopause.

Parmi les symptômes et les conséquences liées à la ménopause, on entend plus précisément les bouffées de chaleur, les sueurs, l'atrophie et la sécheresse vaginale, les symptômes urinaires et à long terme la diminution de la masse osseuse et l'augmentation du risque de fracture, ainsi que la perte de la protection cardio-vasculaire offerte par les oestrogènes.

En particulier, les composés de formule (I) ainsi que leurs sels d'addition avec les acides ou les bases pharmaceutiquement acceptables, peuvent ainsi être utilisés dans la prévention ou le traitement de l'ostéoporose.

Les composés de formule (I) ainsi que leurs sels d'addition avec les acides ou les bases pharmaceutiquement acceptables, peuvent également être utilisés dans la prévention ou le traitement de l'ostéoporos chez l'homme.

Ils peuvent également être utilisés dans la prévention ou le traitement des ostéoporoses secondaires (par exemple cortisoniques ou liées à une immobilisation).

Les composés de formule (I) ainsi que leurs sels d'addition avec les acides ou les bases pharmaceutiquement acceptables possèdent notamment une activité estrogénique dissociée.

Par activité estrogénique dissociée, on entend une activité estrogénique au niveau osseux tout en ne manifestant qu'une activité minimale au niveau utérin entraînant ainsi l'absence de prolifération endométriale (activité bien inférieure à celle de l'oestradiol).

Par ailleurs, les composés selon l'invention présentent les avantages suivants :
- Ils présentent une activité anti-oestrogène et/ou antiproliférative au niveau du sein. A l'opposé de l'oestradiol ils ne stimulent pas la croissance de cellules tumorales mammaires humaines et même peuvent inhiber leur croissance. Les composés selon l'invention sont donc particulièrement avantageux pour le traitement de la ménopause en ce qui concerne les femmes à risque de cancer mammaire (antécédents familiaux) qui sont donc exclues d'un traitement substitutif par l'oestradiol.

Ils peuvent être également utilisables dans le traitement des cancers mammaires.
- Ils entraînent un abaissement du taux de cholestérol sérique à un niveau au moins équivalent à celui induit par l'estradiol. Ils renforcent ainsi la protection cardiovasculaire.
- Enfin les composés selon l'invention ne présentant aucune activité oestrogène au niveau utérin, ne nécessitent pas d'être administrés en association avec un composé progestomimétique.

L'invention a donc pour objet les composés de formule (I) ainsi que leurs sels d'addition avec les acides ou les bases pharmaceutiquement acceptables, à titre de médicaments.

L'invention a plus particulièrement pour objet les composés de formule (I) ainsi que leurs sels d'addition avec les acides ou les bases pharmaceutiquement acceptables, à titre de médicaments destinés à la prévention ou au traitement de l'ostéoporose.

L'invention s'étend aux compositions pharmaceutiques renfermant comme principe actif au moins l'un des médicaments tels que définis ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparation injectables, de pommades, de crèmes, de gels, de microsphères, d'implants, d'anneaux intravaginal, de patches, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie utile varie en fonction de l'affection à traiter et de la voie d'administration ; elle peut varier par exemple de 1 à 1000 mg par jour chez l'adulte par voie orale.

L'invention a encore pour objet l'application de composés de formule (I) tels que définis ci-dessus, pour la préparation d'un médicament destiné au traitement hormonal substitutif de la ménopause ou de la periménopause, ne présentant que peu ou pas d'activité estrogène au niveau utérin et tout particulièrement l'application caractérisée en ce que le médicament est destiné à la prévention ou au traitement de l'ostéoporose.

Les composés de formule (II) ou (IIIₐ) sont des composés connus ou aisément accessible à l'homme du métier. Notamment, les composés de formule (II) avec Z=H, R₂=Me et RA = -Ph-Y-(CH₂)ₙ-Hal sont décrits dans la demande internationale WO 93/13123 (composés de formule II) ; les composés de formule (II) avec Z=H, R₂=Me et RA=-Ph-Y-(CH₂)ₙ-OH sont décrits dans le brevet européen 0305242 B1 (composés de formule (III)), les composés de formule (II) avec Z=H, R₂=Me et RA=-Ph-Y-(CH₂)ₙ-NR₃R₄ sont décrits dans le brevet européen 0097572, le certificat d'addition français 2640977 ou le brevet européen 0305242.

Les composés de formule (II) dans laquelle Z représente un atome d'halogène sont décrits dans la demande internationale WO 9845316 et sont préparés à partir du composé de formule (IV) : 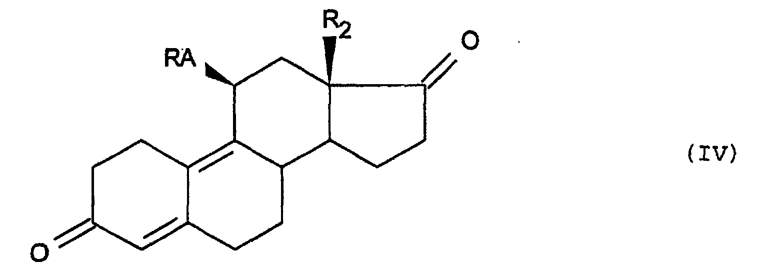 par action d'un réactif d'halogénation afin d'obtenir le composé de formule (V) : 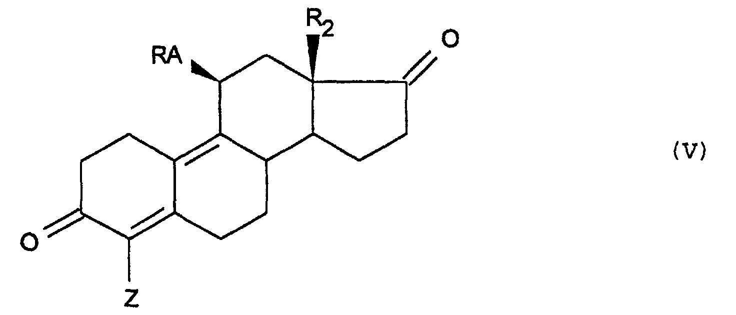 composé (V) que l'on soumet à l'action d'un réactif d'aromatisation du cycle A, puis à l'action d'une base afin d'obtenir le composé de formule (II) dans laquelle Z représente un atome d'halogène.

L'action d'un réactif d'halogénation tel que le N-bromosuccinimide ou le N-chlorosuccinimide sur les composés de formule (IV) s'effectue notamment en présence d'un solvant aprotique dipolaire tel que le diméthylformamide.

La réaction d'aromatisation suivie de la réaction de saponification (action de la base) s'effectue selon les méthodes classiques telles que décrites dans le brevet européen 0097572. On utilise de préférence un mélange d'anhydride acétique et de bromure d'acétyle comme agent d'aromatisation puis une base telle que la soude dans le méthanol comme agent de saponification.

L'invention a également pour objet à titre de produits intermédiaires, les composés de formules (I'ₐ), (III_{b}) et (I'_{b}).

Les exemples ci-dessous illustrent l'invention sans toutefois la limiter.

Solvants décrits dans les exemples : AcOEt (acétate d'éthyle), TEA (triéthylamine), CH₂Cl₂ (dichlorométhane), CHCl₃ (chloroforme), MeOH (méthanol), NH₄OH (hydroxyde d'ammonium), iPrOH (alcool isopropylique).

### EXEMPLE 1 : 17 alpha-fluoro-11 bêta-[4-[2-(1-pipéridinyl) éthoxy]phényl]-estra-1,3,5(10)-trièn-3-ol

### Stade A : Réduction

### 11 bêta-[4-(2-iodoéthoxy)phényl]estra-1,3,5(10)-triène-3,17 bêta-diol

A une solution à 0°C de 516 mg de 3-hydroxy-11 bêta-[4-(2-iodoéthoxy) phényl]estra-1,3,5(10)-trièn-17-one préparé selon la méthode décrite dans WO93/13123 dans 5 ml de MeOH et 5 ml de THF, on ajoute 76 mg de borohydrure de sodium et agite 5 minutes à cette température puis 1 heure 30 à température ambiante. On ajoute ensuite 20 ml d'HCl 0,1N et observe la cristallisation. On obtient 460 mg de produit attendu.
Rf AcOEt/TEA 95/5 = 0,67
RMN (CDCl₃,300 MHz)
7,01 et 6,61(4H,2d,H aromatique en 11) ; 6,79(1H,d,H1) ; 6,68(1H,d,H4) ; 6,51(1H,dd,H2) ; 4,11(2H,t,CH₂O) ; 3,94(1H,tl,H11)) ; 3,72(1H,tl,H17) ; 3,33(2H,t,CH₂I) ; 0,37(3H,s,18-Me).

### Stade B : Fluoration

### Nonafluorobutanesulfonate de 17 alpha-fluoro 11-bêta-[4-(2-iodoéthoxy)phényl]-estra-1,3,5(10)-trièn-3-ol.

A une suspension de 518 mg du stéroïde préparé au stade A dans 5 ml de toluène et 1 ml de dichlorométhane, on ajoute à 0°C et sous azote 0,45 ml de DBU puis 0,20 ml de fluorure de perfluoro-1-butane sulfonyle (FSO₂C₄F₉) et maintient la solution obtenue 30 minutes à température ambiante. On verse ensuite le milieu réactionnel dans 20 ml d'eau, extrait à l'acétate d'éthyle, lave, sèche et évapore sous pression réduite jusqu'à obtention de 565 mg de produit brut que l'on purifie par chromatographie sur Lichrosorb RP18, en éluant avec le mélange acétonitrile/eau 85/15. On obtient 350 mg de produit attendu.
Rf CH₃CN/H₂O 85/15 = 0,47 sur LKC18F (Whatman)
F = 110°C
RMN (CDCl₃,250 MHz)
7,00(2H,m, H1 et H4) ; 6,93 et 6,63(4H, 2d, AA'BB', H aromatiques en 11) ; 6,82(1H,dd, H2) ; 4,44(1H,dd, J₁=55,5 Hz J₂=5Hz, H17) ; 4,14(2H, t, CH₂O) ; 4,03(1H, m, H11) ; 3,35(2H, t, CH₂I) ; 0,23(3H,d, J=2Hz, CH₃).

### Stade C : Introduction de l'amine puis déprotection.

On ajoute 0,4 ml de pipéridine à une solution du stéroïde préparé au stade précédent dans 3 ml de THF, porte l'ensemble au reflux pendant 2 heures puis après refroidissement, verse dans 10 ml d'eau, extrait à l'acétate d'éthyle, lave, sèche et évapore sous pression réduite jusqu'à obtention de 320 mg de produit brut attendu. Après dissolution dans 30 ml de THF, on ajoute 2 ml de fluorure de tétrabutylamonium et chauffe 7 heures au reflux. Après refroidissement, on verse dans 20 ml d'eau saturée de bicarbonate de sodium, extrait à l'acétate d'éthyle, lave, sèche et évapore sous pression réduite.
On obtient 405 mg de produit brut que l'on purifie par chromatographie sur gel de silice, en éluant avec le mélange AcOET/TEA 95/5. On obtient 160 mg de produit pur attendu.
Rf AcOEt/TEA 95/5 0,31 sur SiO₂F₂₅₄ Merck.
RMN (CDCl₃ 300 MHz)
6,95 et 6,48(4H,2d,AA'BB', H aromatiques en 11) ; 6,79 (1H,d,H1) ; 6,48(1H,d,H4) ; 6,39(1H,dd,H2) ; 4,44(1H,dd, J=56Hz et 5Hz,H17) ; 3,99(1H,tl,H11) ; 4,26 et 3,99(2H,2m, CH₂O) ; 1,65 et 1,46(6H,m,CH₂ en γ et β de la pipéridine) ; 1,20 à 3,20(20H,m H squelette + CH₂N et CH₂ en α de la pipéridine) ; 0,22(3H,d,J=2 Hz,CH₃).
IR (CHCl₃)
- OH 3597 cm⁻¹ + associé
- Aromatiques 1608, 1582, 1512, 1503cm⁻¹

### EXEMPLE 2 : Chlohydrate de 17 alpha-fluoro-11-bêta-[4-[2-(1-pipéridinyl)éthoxy]phényl]-estra-1,3,5(10)-trièn-3-ol

A une solution du composé fluoré de l'exemple 1 dans 10 ml d'acétate d'éthyle à 0 +5°C, on ajoute 2 ml d'une solution d'HCl 4N dans l'acétate d'éthyle et observe la cristallisation. On obtient 1,6 g de cristaux blancs.
Rf AcOEt/TEA 95/5 = 0,21 sur SiO₂F₂₅₄ Merck.
F = 216°C
RMN (CDCl₃ 300MHz)
6,95 et 6,43(4H, 2d, AA'BB', H aromatiques en 11) ; 6,79(1H, d, H1) ; 6,66(1H, d, H4) ; 6,59(1H, dd,H2) ; 4,43(1H, dd, J=56 Hz et 5Hz, H17) ; 3,99(1H, tl, H11) ; 4,26 et 3,87(2H, 2m, CH₂O) ; 3,48(1H mobile, NH⁺) ; 2,8 à 3,25 (2H,m, CH₂N⁺) ; 0,22(3H,d,J=2Hz, CH₃).
IR (CHCl₃)
- OH 3598 cm⁻¹ + associé
- absorption NH⁺
- aromatiques 1610, 1582, 1512cm⁻¹

### EXEMPLE 3 : 17 alpha fluoro-11 bêta-[4-[2-(1-diéthylamino) éthoxy]phényl]-estra-1,3,5(10)-trièn-3-ol

On opère comme à l'exemple 1 mais en utilisant la diéthylamine à la place de la pipéridine au cours de l'étape C, on obtient 0,141 g de produit attendu.
Rf AcOEt/Et3N 99/1 = 0,15 sur SiO₂F₂₅₄ Merck.
RMN (CDCl₃ 300 MHz)
0,24(d,CH₃ en 18) ; 1,05(t,CH₃CH₂N) ; 2,64(q,CH₃CH₂N) ; 2,83(t,O-CH₂-CH₂N) ; 3,95(t,CH₂O) ; 4,00(tl,H11) ; 4,44(dd,J=56Hz et 5Hz,H17) ; 6,32(d,H₁), 6,38(dd,H2) ; 6,53(d,H4) ; 6,57 et 6,96(AA'BB'', H aromatiques en 11).

### EXEMPLE 4 : 17 alpha-fluoro-11 bêta-[4-[2-(1-pyrrolidinyl) éthoxy]phényl]-estra-1,3,5(10)-trièn-3-ol

On opère comme à l'exemple 1 mais en utilisant la pyrrolidine à la place de la pipéridine au cours de l'étape C. On obtient 0,049 g de produit attendu.
Rf AcOEt/ET₃N 95/5 = 0,18 sur SiO₂F₂₅₄ Merck.
RMN (CDCl₃ 300 MHz)
0,28(d, CH₃ en 18) ; 2,70(m,pyrrolidine) ; 3,03(t, CH₂N) ; 3,98(t, CH₂O) ; 4,45(dd, H17) : 6,37(dd, H2) ; 6,41(d, H4) ; 6,50 et 6,96(AA'BB', H aromatiques en 11) ; 6,78(d, H1).

### EXEMPLE 5 : 4-chloro-17 alpha-fluoro-11-bêta-[4-[2-(1-pipéridinyl)éthoxy]phényl]-estra-1,3,5(10)-trièn-3-ol

### Stade A : Protection du 3-hydroxy

### 4-chloro-3-[[diméthyl(2,2-diméthyl)éthyl)silyl]oxy]-11 bêta-[4- [2-(1-pipéridinyl)éthoxy]phényl]-estra-1,3,5 (10) -trièn-3-one.

A une solution de 4-chloro-3-hydroxy-11 bêta-[4-[2-(1-pipéridinyl)éthoxy]phényl]-estra-1,3,5,(10)-trièn-3-one préparé par action de NCS sur le dérivé Δ 4-5 9-10, 3-one correspondant, suivie d'une réaction d'aromatisation du cycle A dans 30 ml de CH₂Cl₂, on ajoute 1,3 g de chlorure de t-butyldiméthylsilyle, refroidit à 0°C puis ajoute goutte à goutte 1,13 ml de TEA. Après 5 minutes à 0°C on ramène à température ambiante et maintient 3 heures puis verse dans 100 ml d'eau. Après séchage la phase organique est évaporée sous pression réduite jusqu'à obtention de 4,55 g de produit brut que l'on purifie par chromatographie sur gel de silice en éluant avec le mélange AcOEt/TEA 98/2. On obtient 2,68 g de produit pur attendu.
Rf AcOEt/TEA 90/10 = 0,58 sur SiO₂F₂₅₄ Merck.
IR (CHCl₃)
C=O 1733 cm⁻¹
C-H aromatique 2936 - 2859 cm⁻¹

### Stade B : Réduction

### 4-chloro-3-[[diméthyl-(2,2-diméthyléthyl)silyl]oxy]-11 bêta-[4- [2- (1-pipéridinyl)éthoxy]phényl]-estra-1,3,5(10)-trièn-17 bêta-ol.

On ajoute 189 mg de NaBH₄ à une solution de stéroïde obtenue au stade précédent dans 15 ml de méthanol refroidie au bain de glace, agite 5 minutes à 0+5°C puis 30 minutes à température ambiante. On verse dans 100 ml d'eau, extrait à l'acétate d'éthyle, lave, sèche et évapore sous pression réduite. On obtient 1,5 g de produit attendu.
Rf AcOEt/TEA 90/10 = 0,40 sur SiO₂F₂₅₄ Merck.
Stade C : Fluoration puis déprotection

### - Fluoration

A une solution de 596 mg du stéroïde obtenu au stade précédent dans 5 ml de toluène et 1 ml de CH₂Cl₂ on ajoute sous azote, à température ambiante 0,90 ml de DBU, 0,04 ml de fluorure de perfluorobutane sulfonyle et agite 2 heures. Après avoir versé dans 20 ml d'eau, on extrait avec du dichlorométhane, lave, sèche et évapore sous pression réduite. On obtient 1,23 g de produit brut que l'on purifie par chromatographie sur gel de silice en éluant avec le mélange AcOEt/TEA 98/2. On isole 0,6 g du produit fluoré et protégé en position 3.

### - Déprotection

A une solution du produit fluoré dans 3 ml de THF, on ajoute 2 ml du Bu₄NF, chauffe 4 heures au reflux puis après refroidissement verse dans 20 ml d'eau. Après extraction avec du dichlorométhane, lavage et sèchage, on évapore sous pression réduite jusqu'à obtention d'un extrait sec que l'on purifie par chromatographie sur Lichrosorb RP18 en éluant avec le mélange MeOH/H₂O/TEA 90/9/1. On obtient 65 mg de produit attendu.
Rf MeOH H₂O/TEA 90/9/1 = 0,23 sur LKC18F (Whatman)
RMN (CDCl₃ 300 MHz)
6,91 et 6,61(4H,2d,H aromatique en 11) ; 6,80(1H, d,H1) ; 6,61(1H,d,H2) ; 4,34(1H,dd, J=5Hz et 55,5Hz,H17) ; 3,98(1H,masqué,H11) ; 3,98(2H,t,CH₂O) ; 2,70(2H,t,CH₂N) ; 2,47(4H, m, 2CH₂ en α de l'azote de la pipéridine) ; 1,58(4H,m,2CH₂ en β de l'azote de la pipéridine) ; 1,44(2H, m, 1CH₂ en γ de l'azote de la pipéridine) ; 0,22(3H, d, J=2,5Hz, 18-Me).
IR (CHCl₃)
OH 3537 cm⁻¹
Aromatique 1608, 1581, 1512 cm⁻¹

En opérant comme dans les exemples précédents, on a préparé les produits suivants :

### EXEMPLE 6 : 17-iodo-11bêta-[4-[2-(1-pipéridinyl)-éthoxy]-phényl]-estra-1,3,5(10),16-tétraèn-3-ol.

Rf : 0,33 (AcOEt-TEA 90-10).
IR (CHCl₃)
OH : 3600 cm⁻¹ ; aromatique : 1609, 1580, 1512 cm⁻¹.
RMN (CDCl₃, 300MHz)
0,46 : (s,CH₃ en 18) ; 3,95 : (m,CH₂O et H₁₁) ; 6,11 : (H₁₆) ; 6,36 : (dd,H₂) ; 6,44 : (d,H₄) ; 6,71 : (d,H₁) ; ≃6,50-≃6,94: (aromatique en 11).

### EXEMPLE 7 : 17alpha-fluoro-11bêta-[4-[2-(4-méthyl-1-pipéridinyl)-éthoxyl-phényl]-estra-1,3,5(10)-trièn-3-ol.

Rf : 0,22 (AcOEt-TEA 99-1).
IR (CHCl₃)
OH : 3599 cm⁻¹ ; aromatique : 1610, 1581, 1512 (F) cm⁻¹.
RMN (CDCl₃, 300MHz)
0,26 : (s,CH₃ en 18) ; 0,90 : (d,CH₃ en 4 du pipéridinyl) ; ≃3,97 : (m,CH₂O et H₁₁) ; 4,43 : (dd,H₁₇) ; 6,38 : (dd,H₂) ; 6,46 : (d,H₄) ; 6,80 : (d,H₁) ; ≃6,50- ≃6,95 : (aromatique en 11).

### EXEMPLE 8 : Chlorhydrate de 17alpha-fluoro-11bêta-[4-[2-(4-méthyl-1-pipéridinyl)-éthoxy]-phényl]-estra-1,3,5(10)-trièn-3-ol.

F = 200°C.
Rf : 0,15 (AcOEt-TEA 99-1).
RMN (DMSO, 300MHz)
0,16 : (s,CH₃ en 18) ; 0,90 : (d,CH₃ en 4 du pipéridinyl) ; 3,98 : (sl,H₁₁) ; 4,25 : (sl,CH₂O) ; 4,45 : (dd,H₁₇) ; 6,31 : (dd,H₂) ; 6,48 : (d,J=2 H₄) ; 6,71 : (d,H₁) ; 6,71-7,02 : (aromatique en 11) ; 8,97 (s,OH en 3).

### EXEMPLE 9 : 17alpha-fluoro-3-méthoxy-11bêta-[4-[2-(1-pipéridinyl)-éthoxy]-phényl]-estra-1,3,5(10)-triène.

Rf : 0,23 (MeOH-H₂O-TEA 94-5-1).
IR (CHCl₃)
absence d'OH ; aromatique : 1610, 1578, 1512, 1501 cm⁻¹.
RMN (CDCl₃, 300MHz)
0,22 : (s,CH₃ en 18) ; 1,58 : (m) et 2,47 (m) : pipéridine ; 2,70 : (t, CH₂-N) ; 3,73 : (s,CH₃-O) ; 3,98 : (t,CH₂O) ; 4,43 : (dd,H₁₇) ; 6,50 : (dd,H₂) ; 6,64 : (sl,H₄) ; 6,88 : (d,H₁) ; ≃6,62- ≃6,97 : (aromatique en 11).

### EXEMPLE 10 : 17alpha-fluoro-3-méthoxy-11bêta-[4-[2-(1-pyrrolidinyl)-éthoxy]-phényl]-estra-1,3,5(10)-triène.

F = 132°C.
Rf : 0,21 (MeOH-H₂O-TEA 94-5-1).
IR (CHCl₃)
aromatique : 1610, 1578, 1512, 1501 cm⁻¹.
RMN (CDCl₃, 300MHz)
0,22 : (s,CH₃ en 18); 1,78 : (m) et 2,58 (m) : pyrrolidine ; 2,83 : (t,CH₂-N) ; 3,73 : (s,CH₃-O) ; 3,99 : (t,CH₂O) ; 4,43 : (dd,H₁₇) ; 6,50 : (dd,H₂) ; ≃6,65 : (H₄) ; 6,89 : (d,H₁) ; ≃6,64- ≃6,97 : (aromatique en 11).

### EXEMPLE 11 : 17alpha-fluoro-3-méthoxy-11bêta-[4-[2-(diéthylamino)-éthoxy]-phényl]-estra-1,3,5(10)-triène.

Rf : 0,20 (MeOH-H₂O-TEA 94-5-1).
IR (CHCl₃)
aromatique : 1610, 1578, 1512, 1501 cm⁻¹.
RMN (CDCl₃, 300MHz)
0,22 : (s,CH₃ en 18) ; 1,04 : (t) et 2,61 (q) : N-Et₂ ; 2,82: (t,CH₂-N) ; 3,73 : (s,CH₃-O) ; 3,94 : (t,CH₂O) ; 4,43 : (dd,H₁₇) ; 6,49 : (dd,H₂) ; 6,62 : (d,H₄) ; 6,88 : (d,H₁) ; =6,62- ≃6,96 : (aromatique en 11).

### EXEMPLE 12 : (11bêta)-17-chloro-11-[4- [2- (1-pipéridinyl)-éthoxy]-phényl]-estra-1,3,5(10),16-tétraèn-3-ol.

Rf : 0,25 (CH₂Cl₂-MeOH-NH₄OH 95-5-0,5).
RMN (CDCl₃, 300MHz)
0,60 : (s,CH₃ en 18) ; ≃4,01 : (m,CH₂O et H₁₁) ; 5,60 : (d1,H₁₆) ; 6,38 : (dd,H₂) ; 6,45 : (d,H₄) ; 6,70 : (d,H₁) ; ≃6,50- ≃6,93 : (aromatique en 11).

### EXEMPLE 13 : 17alpha-chloro-11bêta-[4-[2-(1-pipéridinyl)-éthoxy]-phényl]-estra-1,3,5(10)-trièn-3-ol.

Rf : 0,10 (CH₂Cl₂-MeOH-NH₄OH 94-5-0,1).
RMN (CDCl₃, 300MHz)
0,41 : (s,CH₃ en 18) ; ≃3,98 : (m,CH₂O et H₁₁ et H₁₇) ; 6,39 : (dd,H₂) ; 6,48 : (H₄) ; 6,79 : (d,H₁) ; ≃6,48- ≃6,94 : (aromatique en 11).

### EXEMPLE 14 : chlorhydrate de 17-iodo-11bêta-[4-[2-(1-pipéridinyl)-éthoxy]-phényl]-estra-1,3,5(10),16-tétraèn-3-ol.

F = 260°C.
Rf : 0,33 (AcOEt-TEA 90-10).
RMN (DMSO, 300MHz)
0,29 : (s,CH₃ en 18) ; 4,02 : (tl,H₁₁) ; 4,25 : (sl,CH₂O) ; 6,15 : (sl,H₁₆) ; 6,29 : (dd,H₂) ; 6,48 : (d,H₄) ; 6,64 : (d,H₁) ; ≃6,73- ≃7,01 : (aromatique en 11) ; 8,94 ≃ 9,78 : H mobiles.

### EXEMPLE 15 : lactate du 17alpha-fluoro-11bêta-[4-[2-(1-pipéridinyl)-éthoxy]-phényl]-estra-1,3,5(10)-trièn-3-ol.

F = 138°C.
RMN (CDCl₃, 300MHz)
0,22 : (d,CH₃ en 18) ; ≃3,15 ; (CH₂N) ; ≃3,93 : (m,CH₂-O) ; ≃3,98 : (m,H₁₁) ; 4,43 : (dd,H₁₇) ; 6,42 : (dd,H₂) ; 6,78 : (d,H₁) ; ≃6,48- ≃6,97 : (aromatique en 11) ; 1,36 (d) et 4,09 (m) : lactate.

### Tests pharmacologiques

### Effet sur la prolifération de cellules mammaires

L'activité proliférative des molécules est étudiée comparativement à celle de l'oestradiol sur les cellules mammaires humaines MCF-7 en culture.

Pour mettre en évidence un effet agoniste de l'oestradiol et/ou des molécules testées, le milieu de culture d'entretien des cellules (riche en facteurs de croissance et en stéroïdes) est remplacé par un milieu appauvri, entre autres dépourvu de stéroïdes (DMEM supplémenté par 5 % de sérum déstéroïdé et sans rouge de phénol). Les cellules subissent ce sevrage deux jours avant le début de l'essai.

Après 7 jours de culture en présence des produits à étudier, la prolifération cellulaire est évaluée par dosage du DNA. Dans chaque essai, l'effet de l'oestradiol à 10⁻¹⁰M (croissance cellulaire en présence d'oestradiol moins croissance cellulaire en présence du solvant) détermine le 100 % de l'activité agoniste. L'activité des molécules est évaluée en comparaison à ce témoin interne. Les molécules induisant une croissance cellulaire identique à celle observée avec le solvant seul sont classées "inactives", celles induisant une croissance cellulaire inférieure à celle observée avec le solvant sont classées "inhibiteur".

| | ACTIVITE |
|---|---|
| Estradiol | Agoniste |
| Exemple 1 | Inactif |
| Exemple 2 | Inactif |

### Etude de l'impact osseux d'un produit chez la rate femelle ovariectomisée à l'âge de 3 mois.

Les composés sont testés afin de déterminer leur effet sur la masse osseuse et sur l'activité de formation et de résorption dans le modèle de la rate ovariectomisée à l'âge de 3 mois. Les animaux sont traités en préventif.

### Animaux :

| | |
|---|---|
| Espèce | rat |
| Souche | Sprague-Dawley |
| Sexe | femelle |
| Poids | 250 g à 280 g |
| Nbre d'animaux/groupe | 8 |

### Produits :

1 - Produit à tester : Produit de l'exemple 1.
   * véhicule(s) : huile de maïs, méthylcellulose 0,5 %
   * nombre d'administrations : une fois/jour ; 5 jours/semaine pendant 4 semaines
   * voie d'administration : voie orale pour le produit
   * volumes : 5 ml/kg (p.o.)
   * délai entre la dernière injection et le sacrifice : 24 heures
   * nombre d'administrations : 20.
2 - Produit de référence : le 17β oestradiol est administré par voie sous cutanée à la dose 0,1 ou 0,01 mg/kg/j en solution dans un mélange d'huile de germe de maïs-alcool benzylique (99:1, v/v) sous un volume de 0,2 ml/kg.

### Protocole expérimental

### Animaux

L'étude est réalisée chez des rats femelles ovariectomisées à l'âge de 3 mois. Les animaux sont maintenus dans une pièce climatisée (température 20°C ± 2°C) et groupés par 4 dans des boîtes. Les animaux reçoivent, ad libitum, de l'eau déminéralisée et des aliments comprimés (bouchons : AO4CR-10 UAR).

### Chirurgie

Des rats femelles âgés de 3 mois pesant environ 250 g sont ovariectomisés sous anesthésie à l'Imalgène 1000, à la dose de 100 mg/kg par voie intrapéritonéale (i.p.) et sous un volume de 1 ml/kg. Ils reçoivent également du Nembutal (3 mg/kg i.p. sous un volume de 0,3 ml/kg).

Après incision latérale, les plans cutanés et musculaires sont sectionnés. L'exérèse de chaque ovaire se fait après ligature de l'oviducte.

Les rats témoins "SHAM" sont anesthésiés dans les mêmes conditions. Après incision des plans cutanés et musculaires, chaque ovaire est exposé puis replacé in situ.

### Traitement

Les effets des produits sont déterminés en traitement préventif. Ils sont administrés immédiatement après l'ovariectomie. Les animaux répartis en groupes de 8.
Groupe 1 : rats témoins "SHAM" recevant le ou les véhicules
Groupe 2 : rats témoins "OVX" recevant le ou les véhicules
Groupes X : rats "OVX" recevant respectivement les doses définies du ou des produits à tester.

### Prélèvements sanguins

Au terme des 4 semaines (durée de l'étude) les animaux sont décapités par guillotine. Les sérums recueillis après centrifugation sont conservés à -20°C.

Un bilan lipidique sera établi à partir des dosages sériques du cholestérol total, des triglycérides et des phospholipides sur une aliquote de sérum de 500 µl. La baisse du taux de cholestérol sérique est exprimée en % par rapport au taux présenté par les animaux ovariectomisés ne recevant que le solvant.

### Prélèvements d'organes

Après sacrifice des animaux, les organes suivants sont prélevés :
- tractus génital
   Les utérus sont prélevés. Ces derniers sont pesés. L'augmentation du poids est exprimée, en % du poids de l'utérus des animaux ovariectomisés ne recevant que le solvant.
- au niveau osseux :
   La masse osseuse (BMD ou Bone mineral density = densité minérale osseuse) est mesurée par absorptiométrie biphotonique à rayons X en double énergie (DEXA). Les mesures sont réalisées sur les os excisés et débarrassés de tous les tissus mous. La BMD (Bone mineral density) est mesurée sur l'os entier ainsi que sur la partie métaphysaire au niveau de l'extrémité proximale pour le tibia gauche. Cette zone est définie comme étant la région la plus riche en os trabéculaire ; et par conséquent, est la plus sensible aux variations de volume osseux et de densité minérale osseuse.

Les résultats sont exprimés en % selon la formule :$\frac{\text{BMD produit testé - BMD OVX}}{\text{BMD SHAM - BMD OVX}} \text{x 100}$

| | Dose voie mg/kg | OS TIBIA BMD % | UTERUS Poids % | Cholestérol % |
|---|---|---|---|---|
| OVX | | 0 | | |
| SHAM | | 100 | | |
| Estradiol | 0,1 sc | 139 | 220 | -3 |
| Ex. 1 | 0,3 po | 73 | 41 | -51 |
| Estradiol | 0,01 sc | 95 | 317 | -16 |
| Ex. 1 | 0,1 po | 57 | 67 | -49 |
| | 0,3 po | 71 | 70 | -59 |
| | 1,0 po | 72 | 93 | -61 |

## Revendications

1. Composés de formule générale (I) : 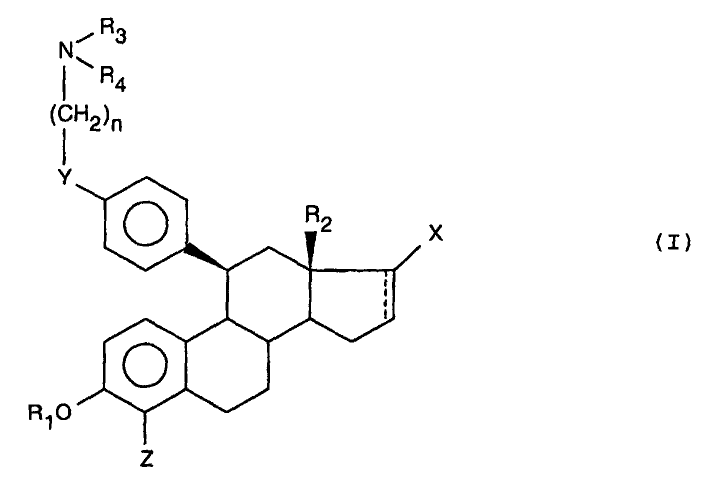 dans laquelle :
R₁ représente un atome d'hydrogène ou un radical choisi parmi méthyle, éthyle, COCH₃, COEt, COPh et CH₂Ph
R₂ représente un radical méthyle,
X représente un atome d'halogène,
Y représente une simple liaison, O, NH, S, SO ou SO₂
Z représente un atome d'hydrogène ou un atome d'halogène
n est égal à 2, 3, 4 ou 5,
soit R₃ et R₄ identiques ou différents représentent un atome d'hydrogène ou un groupement alkyle renfermant de 1 à 6 atomes de carbone,
soit R₃ et R₄ forment ensemble avec l'atome d'azote auquel ils sont liés un hétérocycle mono ou polyclique, saturé, de 3 à 15 chaînons renfermant éventuellement de 1 à 3 hétéroatomes additionnels choisis parmi l'oxygène, le soufre et l'azote, non substitué ou substitué par un méthyle,
les traits en pointillés représentent une seconde liaison éventuelle, ainsi que leurs sels d'addition avec les bases ou les acides.

2. Composés de formule générale (I) telle que définie à la revendication 1, ainsi que leurs sels d'addition, dans laquelle X est un atome de fluor en position alpha, et les traits en pointillés ne représentent pas une seconde liaison.

3. Composés de formule générale (I) telle que définie à la revendication 1 ou 2, ainsi que leurs sels d'addition, dans laquelle R₁ est un atome d'hydrogène, et Z est soit un atome d'hydrogène, soit un atome de chlore, Y représente un atome d'oxygène et les traits en pointillés ne représentent pas une seconde liaison.

4. Composés de formule générale (I) telle que définie à l'une quelconque des revendications 1 à 3, ainsi que leurs sels d'addition, dans laquelle :
soit R₃ et R₄, identiques ou différents, représentent un radical alkyle renfermant de 1 à 6 atomes de carbone,
soit R₃ et R₄ forment ensemble avec l'atome d'azote auquel ils sont liés un des hétérocycles saturés suivants :

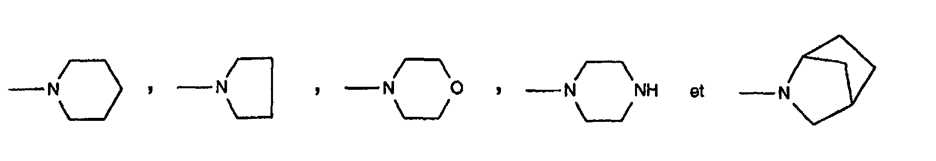

5. Composés de formule générale (I) telle que définie à l'une quelconque des revendications 1 à 4, ainsi que leurs sels d'addition, dans laquelle X est un atome de fluor en position alpha, R₁ est un atome d'hydrogène, R₂ est un radical méthyle, Y est un atome d'oxygène, Z est un atome d'hydrogène ou un atome de chlore, n est égal à 2 ou 3,
soit R₃ et R₄, identiques ou différents, représentent un radical alkyle renfermant de 1 à 6 atomes de carbone,
soit R₃ et R₄ forment ensemble avec l'atome d'azote auquel ils sont liés un des hétérocycles saturés suivants : 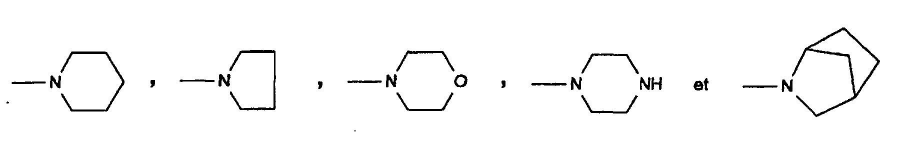 et les traits pointillés ne représentent pas une seconde liaison.

6. Composés selon l'une quelconque des revendications 1 à 5, dont les noms suivent :
- 17 alpha-fluoro-11 bêta-[4-[2-(1-pipéridinyl)éthoxy] phényl]-estra-1,3,5(10)-trièn-3-ol,
- Chlorhydrate de 17 alpha-fluoro-11-bêta-[4-[2-(1-pipéridinyl)éthoxy]phényl]-estra-2,3,5(10) -trièn-3-ol,
- 17 alpha-fluoro-11 bêta-[4-[2-(1-diéthylamino) éthoxy] phényl]-estra-1,3,5(10)-trièn-3-ol,
- 17 alpha-fluoro-11 beta-[4-[2-(1-pyrrolidinyl) éthoxy] phényl]-estra-1,3,5(10)-trièn-3-ol,
- 4-chloro-17 alpha-fluoro-11-bêta- [4- [2- (1-pipéridinyl) éthoxy]phényl]-estra-1,3,5(10)-trièn-3-ol,
- 17-iodo-11 bêta-[4-[2-(1-pipéridinyl)éthoxy]phényl]-estra-1,3,5(10),16-tétraèn-3-ol,
- 17alpha-fluoro-11bêta-[4-[2-(4-méthyl-1-pipéridinyl)-éthoxy]-phényl]-estra-1,3,5(10)-trien-3-ol,
- chlorhydrate de 17alpha-fluoro-11bêta-[4-[2-(4-méthyl-1-pipéridinyl)-éthoxy]-phényl]-estra-1,3,5(10)-trien-3-ol,
- 17alpha-fluoro-3-méthoxy-11bêta-[4-[2-(1-pipéridinyl)-éthoxy]-phényl]-estra-1,3,5(10)-triène,
- 17alpha-fluoro-3-méthoxy-11bêta-[4-[2-(1-pyrrolidinyl)-éthoxy]-phényl]-estra-1,3,5(10)-triène,
- 17alpha-fluoro-3-méthoxy-11bêta- [4- [2-(diéthylamino)-éthoxy]-phényl]-estra-1,3,5(10)-triène,
- (11bêta)-17chloro-11-[4-[2-(1-pipéridinyl)-éthoxy]-phényl]-estra-1,3,5(10),16-tétraèn-3-ol,
- 17alpha-chloro-11bêta-[4-[2-(1-pipéridinyl)-éthoxy]-phényl]-estra-1,3,5(10)-trièn-3-ol,
- chlorhydrate de 17-iodo-11bêta-[4-[2-(1-pipéridinyl)-éthoxy]-phényl]-estra-1,3,5(10),16-tétraèn-3-ol,
- lactate de 17alpha-fluoro-11bêta-[4-[2-(1-pipéridinyl)-éthoxy]-phényl]-estra-1,3,5(10)-trièn-3-ool.

7. Procédé de préparation des composés de formule générale (I) telle que définie à la revendication 1, **caractérisé en ce que** l'on soumet un composé de formule (II) : 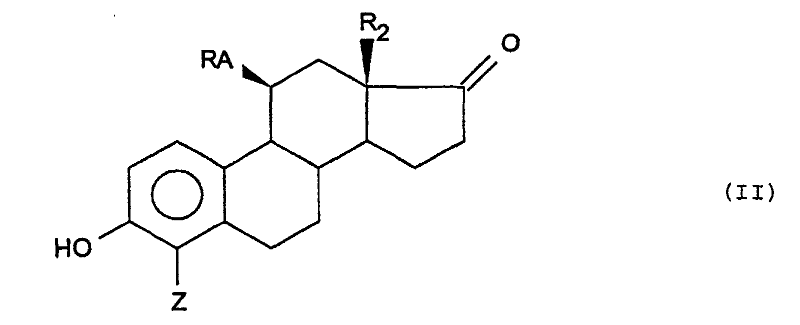 dans laquelle :
R₂ et Z sont tels que définis précédemment,
RA représente un des groupements suivants : 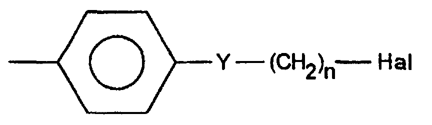 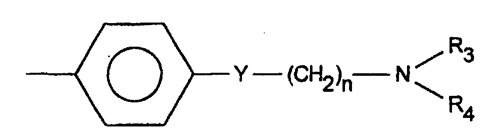 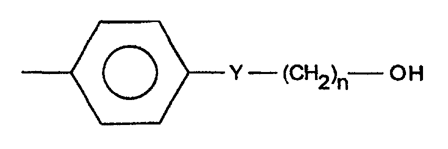 dans lesquels Y, n, R₃ et R₄ sont tels que définis précédemment et Hal représente un atome d'halogène, à l'action, après le cas échéant protection et/ou activation des fonctions OH,
soit a) d'un agent réducteur du céto en 17 afin d'obtenir un composé de formule (IIIₐ) : 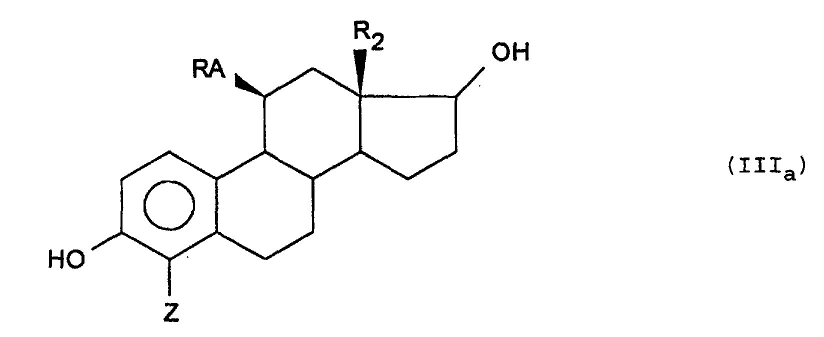
b) puis d'un agent d'halogénation afin d'obtenir un composé de formule (I'ₐ) : 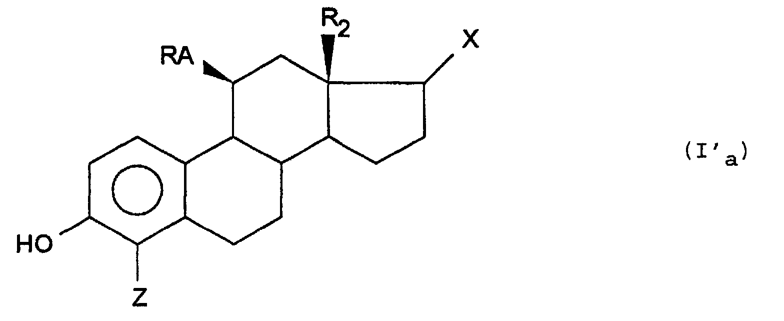
correspondant à certains composés de formule (I), lorsque RA représente -Ph-Y-(CH₂)ₙ-NR₃R₄,
soit a) d'une hydrazine afin d'obtenir un composé de formule (III_{b}) : 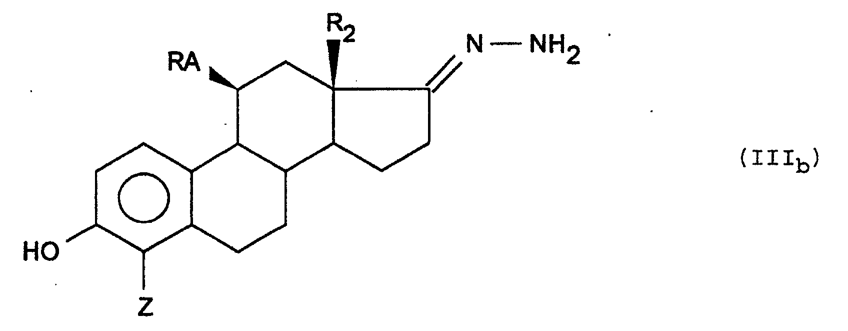
b) puis d'un agent d'halogénation afin d'obtenir un composé de formule (I'_{b}) : 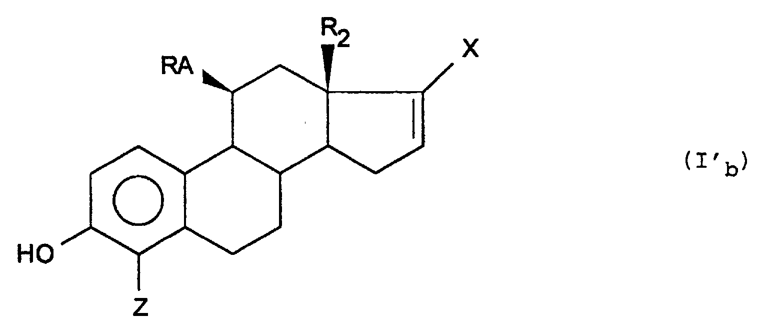
correspondant à certains composés de formule (I), lorsque RA représente -Ph-Y-(CH₂)ₙ-NR₃R₄,
les composés de formule (II), (IIIₐ), (III_{b}), (I'ₐ) ou (I'_{b}) sous forme protégée ou non, étant soumis si désiré ou si nécessaire, à l'une ou plusieurs des réactions suivantes :
- déprotection du ou des groupements OH protégés,
- acylation/alkylation du ou des groupements OH,
- action de HNR₃R₄, éventuellement sous forme de sel, lorsque RA représente le groupement -Ph-Y-(CH₂)ₙ-Hal ou -Ph-Y-(CH₂)ₙ-OH activé,
- salification.

8. Procédé de préparation selon la revendication 7, des composés de formule générale (I) telle que définie à la revendication 2 avec X représentant un atome de fluor en position 17 alpha et les traits en pointillés ne représentent pas une seconde liaison, **caractérisé en ce que** le réactif d'halogénation est le fluorure de perfluoro-1-butane sulfonyle (FSO₂C₄F₉).

9. A titre de médicaments les composés de formule (I) telle que définie à la revendication 1, ainsi que leurs sels d'addition avec les acides ou les bases pharmaceutiquement acceptables.

10. A titre de médicaments les composés de formule (I) telle que définie à l'une quelconque des revendications 2 à 5, ainsi que leurs sels d'addition avec les acides pharmaceutiquement acceptables.

11. A titre de médicaments les composés tels que définis à la revendication 6, ainsi que leurs sels d'addition avec les acides pharmaceutiquement acceptables.

12. Compositions pharmaceutiques renfermant un ou plusieurs des médicaments tels que définis à l'une quelconque des revendications 9, 10 ou 11.

13. A titre de produits intermédiaires nouveaux, les composés de formules générales (I'ₐ), (III_{b}) et (I'_{b}), telles que définies à la revendication 7 ou 8.

14. Application de composés de formule (I) tels que définis aux revendications 1 à 6, pour la préparation d'un médicament destiné au traitement hormonal substitutif de la ménopause ou de la périménopause, ne présentant que peu ou pas d'activité estrogène au niveau utérin.

15. Application selon la revendication 14, **caractérisée en ce que** le médicament est destiné à la prévention ou au traitement de l'ostéoporose.

16. Médicament, tel que défini à la revendication 11, destiné à la prévention ou au traitement de l'ostéoporose.

17. Médicament, tel que défini à la revendication 11, destiné à la protection cardiovasculaire.

## Claims

1. Compounds of general formula (I): 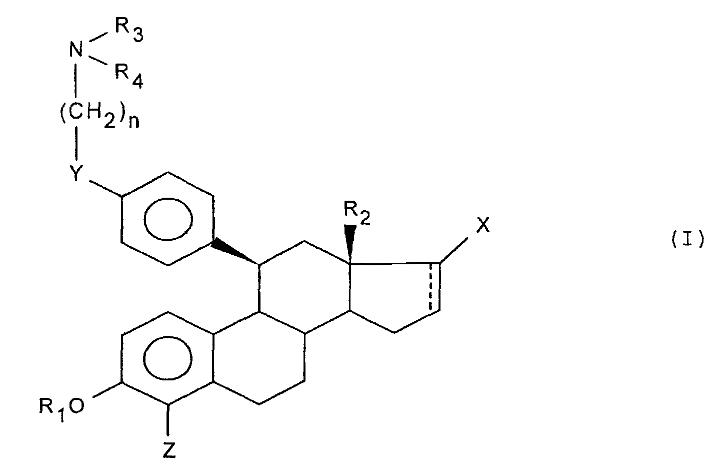 in which:
R₁ represents a hydrogen atom or a radical chosen from methyl, ethyl, COCH₃, COEt, COPh and CH₂Ph,
R₂ represents a methyl radical,
X represents a halogen atom,
Y represents a single bond, O, NH, S, SO or SO₂,
Z represents a hydrogen atom or a halogen atom
n is equal to 2, 3, 4 or 5,
either R₃ and R₄, identical or different, represent a hydrogen atom or an alkyl group containing 1 to 6 carbon atoms,
or R₃ and R₄ form together with the nitrogen atom to which they are linked a saturated, mono- or polycyclic heterocycle with 3 to 15 members optionally containing 1 to 3 additional heteroatoms chosen from oxygen, sulphur and nitrogen, non-substituted or substituted by a methyl, The dotted lines representing an optional second bond, as well as their addition salts with bases or acids.

2. Compounds of general formula (I) as defined in claim 1, as well as their addition salts, in which X is a fluorine atom in alpha position and the dotted lines do not represent a second bond.

3. Compounds of general formula (I) as defined in claim 1 or 2, as well as their addition salts, in which R₁ is a hydrogen atom, and Z is either a hydrogen atom or chlorine atom, Y represents an oxygen atom and the dotted lines do not represent a second bond.

4. Compounds of general formula (I) as defined in any one of claims 1 to 3, as well as their addition salts, in which:
either R₃ and R₄, identical or different, represent an alkyl radical containing 1 to 6 carbon atoms,
or R₃ and R₄ together with the nitrogen atom to which they are linked form one of the following saturated heterocycles:

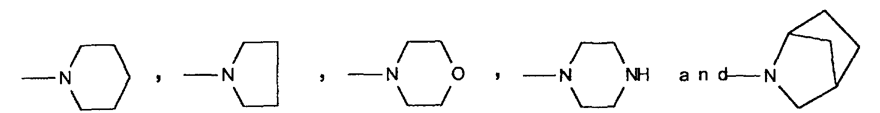

5. Compounds of general formula (I) as defined in any one of claims 1 to 4, as well as their addition salts, in which X is a fluorine atom in alpha position, R₁ is a hydrogen atom, R₂ is a methyl radical, Y is an oxygen atom, Z is a hydrogen atom or chlorine atom, n is equal to 2 or 3,
either R₃ and R₄, identical or different, represent an alkyl radical containing 1 to 6 carbon atoms,
or R₃ and R₄ together with the nitrogen atom to which they are linked form one of the following saturated heterocycles: 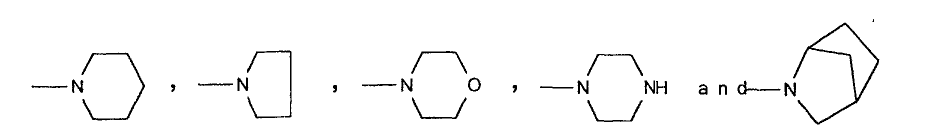 and the dotted lines do not represent a second bond.

6. Compounds according to any one of claims 1 to 5, the names of which follow:
- 17-alpha-fluoro-11-beta-[4-[2-(1-piperidinyl)ethoxy] phenyl)-estra-1,3,5(10)-trien-3-ol
- 17-alpha-fluoro-11-beta-[4-[2-(1-piperidinyl)ethoxy] phenyl]-estra-1,3,5(10)-trien-3-ol hydrochloride
- 17-alpha-fluoro-11-beta-[4-[2-(1-diethylamino)ethoxy] phenyl]-estra-1,3,5(10)-trien-3-ol
- 17-alpha-fluoro-11-beta-[4-[2-(1-pyrrolidinyl)ethoxy] phenyl]-estra-1,3,5(10)-trien-3-ol
- 4-chloro-17-alpha-fluoro-11-beta-[4-[2-(1-piperidinyl) ethoxy] phenyl]-estra-1,3,5(10)-trien-3-ol
- 17-iodo-11-beta-[4-[2-(1-piperidinyl)ethoxy] phenyl]-estra-1,3,5(10),16-tetraen-3-ol,
- 17-alpha-fluoro-11-beta-[4-[2-(4-methyl-1-piperidinyl)-ethoxy]-phenyl]-estra-1,3,5(10)-trien-3-ol,
- 17-alpha-fluoro-11-beta-[4-[2-(4-methyl-1-piperidinyl)-ethoxy]-phenyl]-estra-1,3,5(10)-trien-3-ol hydrochloride,
- 17-alpha-fluoro-3-methoxy-11-beta-[4-[2-(1-piperidinyl)-ethoxy]-phenyl]-estra-1,3,5(10)-triene,
- 17-alpha-fluoro-3-methoxy-11-beta-[4-[2-(1-pyrrolidinyl)-ethoxy]-phenyl)-estra-1,3,5(10)-triene,
- 17-alpha-fluoro-3-methoxy-11-beta-[4-[2-(diethylamino)-ethoxy]-phenyl]-estra-1,3,5(10)-triene,
- (11-beta)-17-chloro-11-[4-[2-(1-piperidinyl)-ethoxy]-phenyl]-estra-1,3,5(10),16-tetraen-3-ol,
- 17-alpha-chloro-11-beta-[4-[2-(1-piperidinyl)-ethoxy]-phenyl]-estra-1,3,5(10)-trien-3-ol,
- 17-iodo-11beta-[4-[2-(1-piperidinyl)-ethoxy]-phenyl]-estra-1,3,5(10),16-tetraen-3-ol hydrochloride,
- 17-alpha-fluoro-11beta-[4-[2-(1-piperidinyl)-ethoxy]-phenyl]-estra-1,3,5(10)-trien-3-ol lactate.

7. A preparation process for the compounds of general formula (I) as defined in claim 1, **characterized in that** a compound of formula (II): 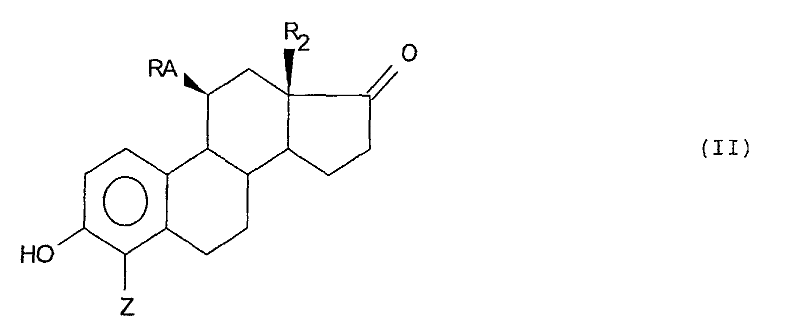 in which
R₂ and Z are as defined previously,
RA represents one of the following groups: 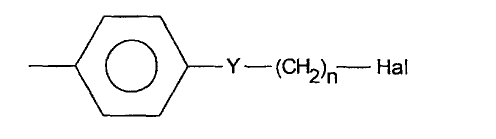 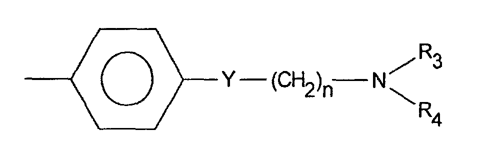 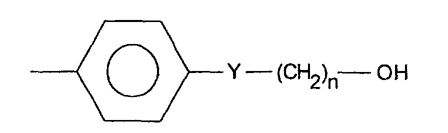 in which Y, n, R₃ and R₄ are as defined previously and Hal represents a halogen atom, is subjected to the action, if appropriate after protection and/or activation of the OH functions,
either a) of a reducing agent of the keto in position 17 in order to obtain a compound of formula (IIIₐ): 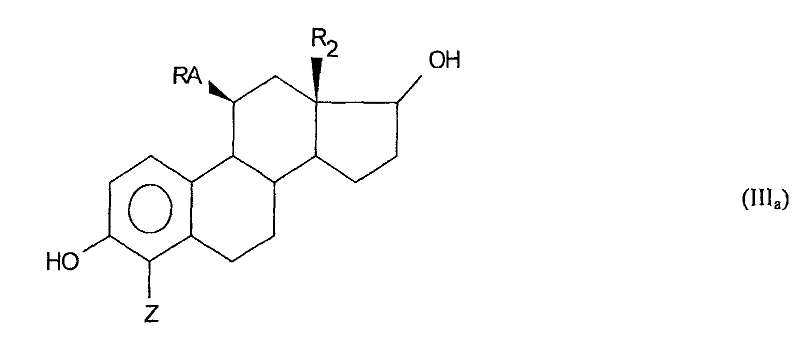
b) then of a halogenation agent in order to obtain a compound of formula (I'ₐ): 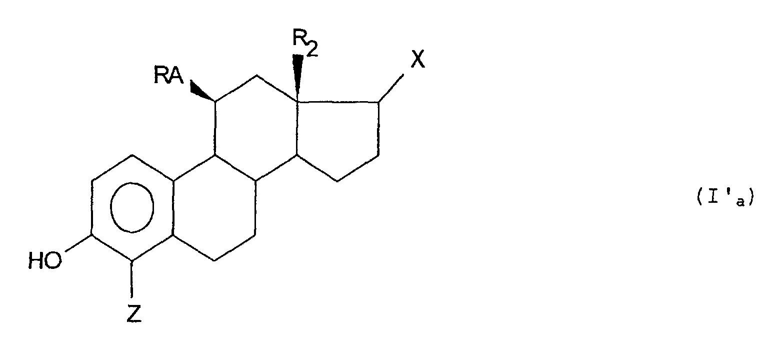
corresponding to certain compounds of formula (I), when RA represents -Ph-Y-(CH₂)ₙ-NR₃R₄,
or a) of a hydrazine in order to obtain a compound of formula (III_{b}): 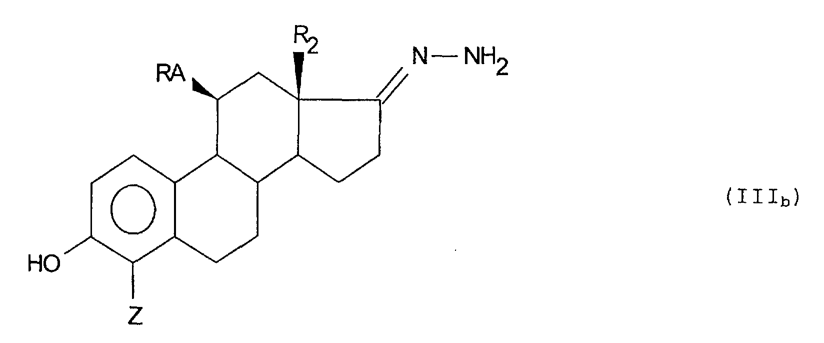
b) then of a halogenation agent in order to obtain a compound of formula (I'_{b}): 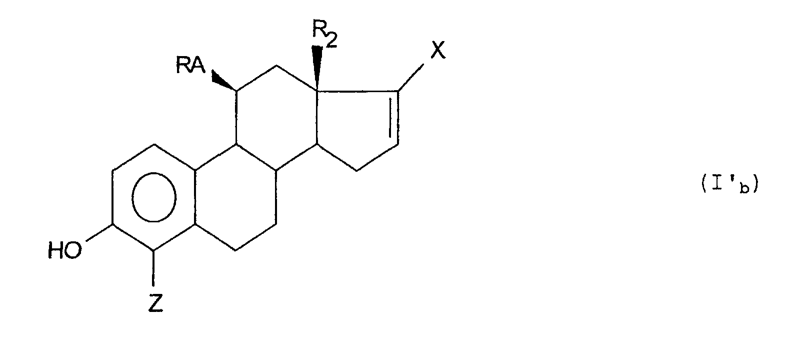
corresponding to certain compounds of formula (I), when RA represents -Ph-Y-(CH₂)ₙ-NR₃R₄,
The compounds of formula (II), (IIIₐ), (III_{b}), (I'ₐ) or (I'_{b}), in protected or unprotected form, being subjected, if desired or if necessary, to one or more of the following reactions:
- deprotection of the protected OH group or groups,
- acylation/alkylation of the OH group or groups,
- the action of HNR₃R₄, optionally in the form of a salt, when RA represents the -Ph-Y-(CH₂)ₙ-Hal or activated -Ph-Y-(CH₂)ₙ-OH group,
- salification.

8. Preparation process according to claim 7 for compounds of general formula (I) as defined in claim 2 with X representing a fluorine atom in position 17 alpha and the dotted lines do not represent a second bond, **characterized in that** the halogenation reagent is perfluoro-1-butane sulphonyl fluoride (FSO₂C₄F₉).

9. As medicaments, the compounds of formula (I) as defined in claim 1, as well their addition salts with pharmaceutically acceptable acids or bases.

10. As medicaments the compounds of formula (I) as defined in any one of claims 2 to 5, as well as their addition salts with pharmaceutically acceptable acids.

11. As medicaments the compounds as defined in claim 6, as well as their addition salts with pharmaceutically acceptable acids.

12. Pharmaceutical compositions containing one or more medicaments as defined in any one of claims 9, 10 or 11.

13. As new intermediate products, the compounds of general formulae (I'ₐ), (III_{b}) and (I'_{b}), as defined in claim 7 or 8.

14. Use of compounds of formula (I) as defined in claims 1 to 6, for the preparation of a medicament intended for hormone replacement treatment for the menopause or the perimenopause, having little or no activity at the uterine level.

15. Use according to claim 14, **characterized in that** the medicament is intended for the prevention or treatment of osteoporosis.

16. Medicament, as defined in claim 11, intended for the prevention or treatment of osteoporosis.

17. Medicament, as defined in claim 11, intended for cardiovascular protection.

## Patentansprüche

1. Verbindungen der allgemeinen Formel (I): 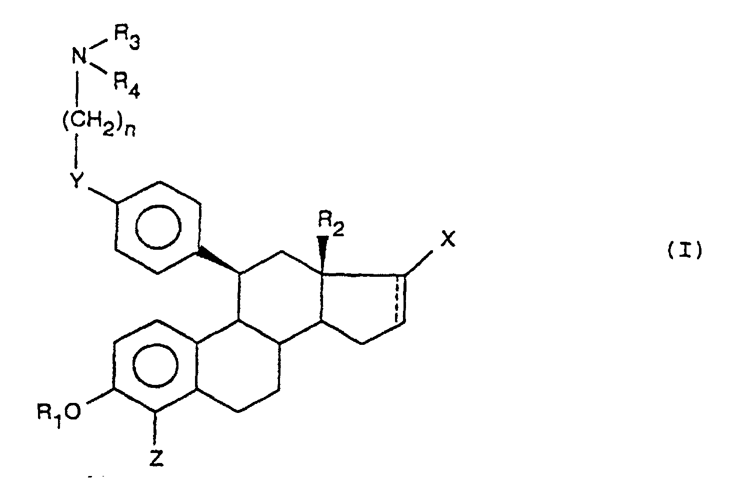 in der:
R₁ ein Wasserstoffatom oder eine Gruppe, ausgewählt aus Methyl, Ethyl, COCH₃, COEt, COPh und CH₂Ph, darstellt;
R₂ eine Methyl-Gruppe darstellt;
X ein Halogenatom darstellt;
Y eine Einfachbindung, O, NH, S, SO oder SO₂ darstellt;
Z ein Wasserstoffatom oder ein Halogenatom darstellt;
n für 2, 3, 4 oder 5 steht,
R₃ und R₄, die gleich oder unterschiedlich sind, ein Wasserstoffatom oder ein Alkylgruppierung, die 1 bis 6 Kohlenstoffatome umfaßt, darstellen
oder R₃ und R₄ zusammen mit dem Stickstoffatom, an das sie gebunden sind, einen gesättigten monocyclischen oder polycyclischen Heterocyclus mit 3 bis 15 Kettengliedern bilden, der gegebenenfalls 1 bis 3 zusätzliche Heteroatome, ausgewählt aus Sauerstoff, Schwefel und Stickstoff, durch ein Methyl substituiert oder nicht-substituiert, umfaßt, wobei die punktierte Linie eine mögliche zweite Bindung darstellt,
sowie ihre Additionssalze mit Basen oder Säuren.

2. Verbindungen der allgemeinen Formel (I) nach Anspruch 1 sowie ihre Additionssalze, wobei X ein Fluoratom in alpha-Position ist und die punktierte Linie keine zweite Bindung darstellt.

3. Verbindungen der allgemeinen Formel (I) nach Anspruch 1 oder Anspruch 2 sowie ihre Additionssalze, wobei R₁ ein Wasserstoffatom ist und Z entweder ein Wasserstoffatom oder ein Chloratom ist, Y ein Sauerstoffatom darstellt und die punktierte Linie keine zweite Bindung darstellt.

4. Verbindungen der allgemeinen Formel (I) nach einem der Ansprüche 1 bis 3 sowie ihrer Additionssalze, wobei
R₃ und R₄, die gleich oder unterschiedlich sind, eine Alkyl-Gruppe, die 1 bis 6 Kohlenstoffatome hat, darstellen;
oder R₃ und R₄ zusammen mit dem Stickstoffatom, an das sie gebunden sind, einen der folgenden gesättigten Heterocyclen bilden:

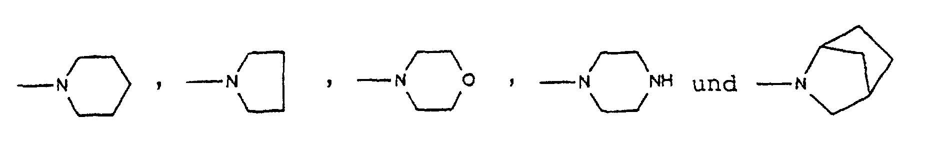

5. Verbindungen der allgemeinen Formel (I) nach einem der Ansprüche 1 bis 4 sowie ihre Additionssalze, wobei X ein Fluoratom in alpha-Position ist, R₁ ein Wasserstoffatom ist, R₂ eine Methyl-Gruppe ist, Y ein Sauerstoffatom ist, Z ein Wasserstoffatom oder ein Chloratom ist, n 2 oder 3 ist,
R₃ und R₄, die gleich oder unterschiedlich sind, eine Alkyl-Gruppe, die 1 bis 6 Kohlenstoffatome umfaßt, darstellen,
oder R₃ und R₄ zusammen mit dem Stickstoffatom, an das sie gebunden sind, einen der folgenden gesättigten Heterocyclen bilden: 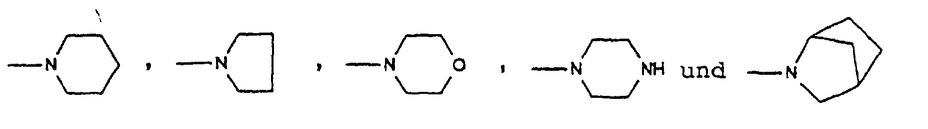 und die gepunktete Linie keine zweite Bindung darstellt.

6. Verbindungen nach einem der Ansprüche 1 bis 5 mit den folgenden Namen:
17-alpha-Fluor-11-beta-[4-[2-(1-piperidinyl)-ethoxy]phenyl]-estra-1,3,5(10)-trien-3-ol,
Hydrochlorid von 17-alpha-Fluor-11-beta-[4-[2-(1-piperidinyl)ethoxy]phenyl]-estra-1,3,5(10)-trien-3-ol-,
17-alpha-Fluor-11-beta-[4-[2-(1-diethylamino)-ethoxy]phenyl]-estra-1,3,5(10)-trien-3-ol,
17-alpha-Fluor-11-beta-[4-[2-(1-pyrrolidinyl)-ethoxy]phenyl]-estra-1,3,5(10)-trien-3-ol,
4 -Chlor-17-alpha-fluor-11-beta-[4-[2-(1-piperidinyl)ethoxy]phenyl]-estra-1,3,5(10)-trien-3-ol,
17-Iod-11-beta-[4-[2-(1-piperidinyl)ethoxy]phenyl]-estra-1,3,5(10),16-tetraen-3-ol,
17-alpha-Fluor-11-beta-[4-[2-(4-methyl-1-piperidinyl)ethoxy]phenyl]-estra-1,3,5(10)-trien-3-ol,
Hydrochlorid von 17-alpha-Fluor-11-beta-[4-[2-(4-methyl-1-piperidinyl)ethoxy]phenyl]-estra-1,3,5(10)-trien-3-ol,
17-alpha-Fluor-3-methoxy-11-beta-[4-[2-(1-piperidinyl)ethoxy]phenyl]-estra-1,3,5(10)-trien,
17-alpha-Fluor-3-methoxy-11-beta-[4-[2-(1-pyrrolidinyl)ethoxy]phenyl]-estra-1,3,5(10)-trien,
17-alpha-Fluor-3-methoxy-11-beta-[4-[2-(1-diethylamino)ethoxy]phenyl]-estra-1,3,5(10)-trien,
(11-beta)-17-Chlor-11-[4-[2-(1-piperidinyl)-ethoxy]phenyl]-estra-1,3,5,(10),16-tetraen-3-ol,
17-alpha-Clor-11-beta-[4-[2-(1-piperidinyl)-ethoxy]phenyl]-estra-1,3,5(10)-trien-3-ol,
Hydrochlorid von 17-Iod-11-beta-[4-[2-(1-piperidinyl)ethoxy]phenyl]-estra-1,3,5(10),16-tetraen-3-ol,
Lactat von 17-alpha-Fluor-11-beta-[4-[2-(1-piperidinyl)ethoxy]phenyl]-estra-1,3,5(10)-trien-3-ol.

7. Verfahren zur Herstellung der Verbindungen der allgemeinen Formel (I) nach Anspruch 1, **dadurch gekennzeichnet, daß** man eine Verbindung der Formel (II): 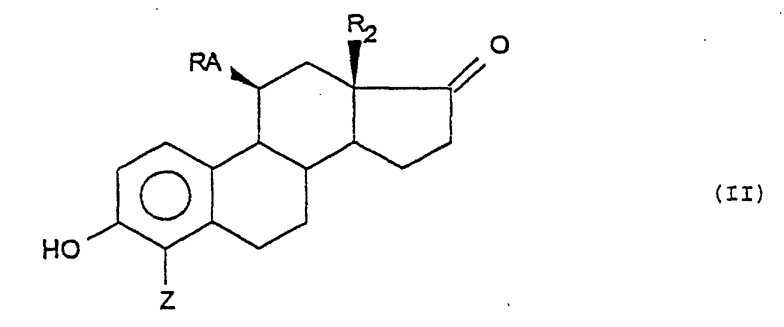 worin:
R₂ und Z die oben gegebenen Definitionen besitzen,
RA eine der folgenden Gruppen darstellt: 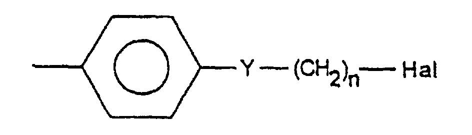 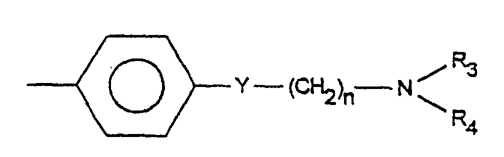 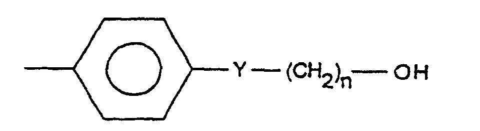 in denen Y, n, R₃ und R₄ die vorstehend gegebenen Bedeutungen besitzen und Hal ein Halogenatom darstellt,
gegebenenfalls nach Schutz und/oder Aktivierung der OH-Funktionen, entweder der Wirkung a) eines Reduktionsmittels für die Keto-Funktion in Position 17,
unterwirft, um eine Verbindung der Formel (IIIₐ) zu erhalten: 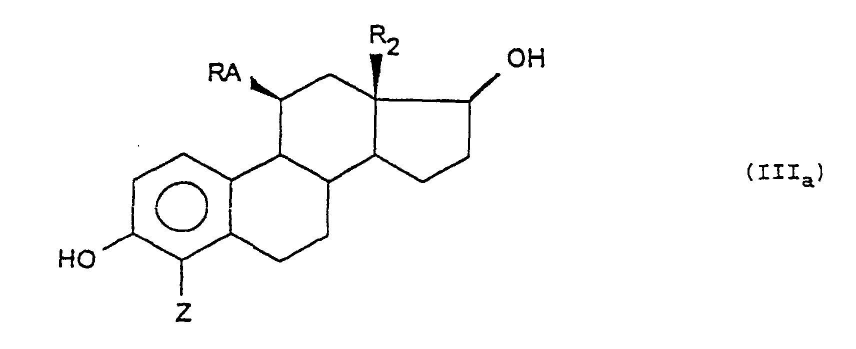 b) dann der Wirkung eines Halogenierungsmittels unterwirft, um eine Verbindung der Formel (I'ₐ) zu erhalten: 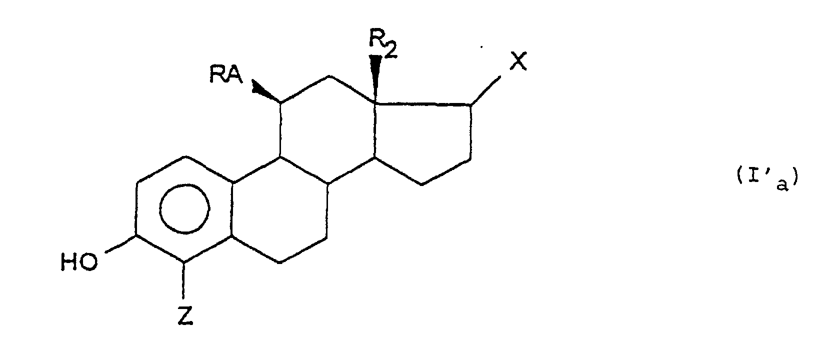 welche bestimmten Verbindungen der Formel (I) entspricht, wenn RA -Ph-Y-(CH₂)ₙ-NR₃R₄ darstellt, unterwirft
oder a) der Wirkung eines Hydrazins unterwirft, um eine Verbindung der Formel (III_{b}) zu erhalten: 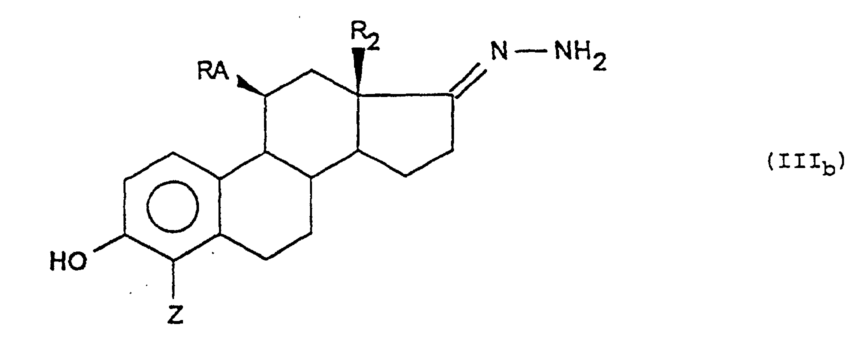
b) dann der Wirkung eines Halogenierungsmittels unterwirft, um eine Verbindung der Formel (I'_{b}) zu erhalten: 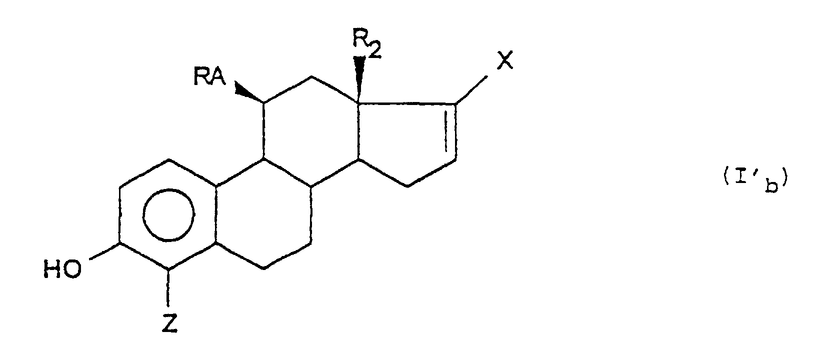
die bestimmten Verbindungen der Formel (I) entspricht, wenn RA -Ph-Y-(CH₂)ₙ-NR₃R₄ entspricht,
die Verbindungen der Formel (II), (IIIₐ), (III_{b}), (I'ₐ) oder (I'_{b}) in geschützter Form oder nicht-geschützter Form auf Wunsch oder bei Bedarf einer oder mehreren der folgenden Reaktionen unterworfen werden:
- Entfernung des Schutzes an der geschützten OH-Gruppierung oder den geschützten OH-Gruppierungen,
- Acylierung/Alkylierung der OH-Gruppierung(en),
- Behandlung mit HNR₃R₄, gegebenenfalls in Salzform, wenn RA die Gruppierung -Ph-Y-(CH₂)ₙ-Hal oder aktiviertes -Ph-Y-(CH₂)ₙ-OH darstellt,
- Salzbildung.

8. Verfahren nach Anspruch 7 zur Herstellung der Verbindungen der allgemeinen Formel (I) nach Anspruch 2, wobei X ein Fluoratom in Position 17 alpha darstellt und die gepunktete Linie keine zweite Bindung darstellt, **dadurch gekennzeichnet, daß** das Halogenierungsreagens Perfluor-1-butansulfonylfluorid (FSO₂C₄F₉) ist.

9. Die Verbindungen der Formel (I) nach Anspruch 1 sowie ihre Additionssalze mit den pharmazeutisch annehmbaren Säuren oder Basen als Arzneimittel.

10. Die Verbindungen der Formel (I) nach einem der Ansprüche 2 bis 5 sowie ihre Additionssalze mit den pharmazeutisch annehmbaren Säuren als Medikamente.

11. Die Verbindungen nach Anspruch 6 sowie ihre Additionssalze mit den pharmazeutisch annehmbaren Säuren als Medikamente.

12. Pharmazeutische Zusammensetzungen, die ein Medikament oder mehrere Medikamente nach einem der Ansprüche 9, 10 oder 11 umfassen.

13. Verbindungen der allgemeinen Formeln (I'ₐ), (III_{b}) und (I'_{b}) nach Anspruch 7 oder Anspruch 8 als neue Zwischenprodukte.

14. Verwendung der Verbindungen der Formel (I) nach Anspruch 1 bis 6 zur Herstellung eines Medikaments, das zur Hormonsubstitutionsbehandlung der Menopause oder der Permenopause bestimmt ist, das wenig oder keine Estrogen-Aktivität auf uterinem Niveau zeigt.

15. Verwendung nach Anspruch 14, **dadurch gekennzeichnet, daß** das Medikament zur Prävention oder Behandlung von Osteoporose bestimmt ist.

16. Medikament nach Anspruch 11, das zur Prävention oder Behandlung von Osteoporose bestimmt ist.

17. Medikament nach Anspruch 11, das zum kardiovaskulären Schutz bestimmt ist.
